# EUROPEAN PATENT SPECIFICATION

(11) **EP 3 520 678 B1**
(45) Date of publication and mention of the grant of the patent: **09.04.2025**
(21) Application number: 19154995.5
(22) Date of filing: 01.02.2019
(51) Int. Cl.: A61B 3/00, A61B 3/032

(54) **SUBJECTIVE OPTOMETRY APPARATUS**
SUBJEKTIVE OPTOMETRIEVORRICHTUNG
APPAREIL D'OPTOMÉTRIE SUBJECTIVE

(30) Priority: 02.02.2018 JP 2018016847
(43) Date of publication of application: 07.08.2019
(73) Proprietor: Nidek Co., Ltd., Gamagori-Aichi, 443-0038 (JP)
(72) Inventor: TAKII, Michihiro, Gamagori, Aichi (JP); TACHIBANA, Sasagu, Gamagori, Aichi (JP)
(74) Representative: Hoefer & Partner Patentanwälte mbB

(56) References cited:
- EP-A1- 3 150 111
- WO-A2-2007/026368
- JP-A- 2017 099 640

## Description

### TECHNICAL FIELD

The present disclosure relates to a subjective optometry apparatus which subjectively measures optical characteristics of a subject eye.

### BACKGROUND

As a subjective optometry apparatus, an apparatus which measures optical characteristics (eye refractive power) of the subject eye by disposing an optical member (for example, a spherical lens, a cylindrical lens, and the like) in front of the eyes of an examinee and presenting an examination visual target via the optical member is known (refer to JP-AH05-176893).

From WO 2007/026368 A2, a multi-functional optometricophthalmic system for testing, diagnosing, or treating, vision or eyes of a subject, and methodologies thereof is known, which includes: a head mountable unit, including a head mounting assembly, and at least one near eye module assembly, mounted upon the head mounting assembly, for generating optical processes or effects which act or take place upon, and are affected by, at least one eye of the subject, and for receiving results of the optical processes or effects from the at least one eye, as part of the testing, diagnosing, or treating of the vision or eyes of the subject; and a central controlling and processing unit. Therein, the near eye module assembly includes: a micro-display, a first lens assembly, and a refraction correction assembly.

From EP 3 150 111 A1, an ophthalmic apparatus for examining an eye of an examinee is known which comprises: optometry means configured to examine the examinee's eye; drive means for relatively moving the optometry means three-dimensionally with respect to the examinee's eye; first photographing means configured to photograph a face of the examinee including at least one of right and left eyes of the examinee; and control means configured to control the drive means. The control means is further configured to restrict a drive range of the drive means based on a two-dimensional position of the examinee's eye on a first image photographed by the first photographing means, the two-dimensional position being specified by image processing on the first image, and information on the first photographing means.

However, in subjective measurement, it is necessary to project the examination visual targets having various angles of view onto the subject eye in accordance with the examination contents. As an example, there is a case where a Landolt ring visual target displayed on a display or the like is projected onto the subject eye in order to measure the visual acuity value. Further, as an example, there is a case where a landscape visual target displayed on the display or the like is projected onto the subject eye in order to confirm the appearance in everyday life. However, the size of the examination visual target that can be displayed is limited due to the screen size and the pixel density of the display. Therefore, the examination visual target which can be projected onto the subject eye has a maximum angle of view and a minimum angle of view, and there is a case where it is not possible to respond to a case where it is desired to project the examination visual target onto the subject eye at an angle of view which is out of the range.

### SUMMARY

An object of the present disclosure is to provide a subjective optometry apparatus which can further widen a variable angle-of-view range of an examination visual target projected to a subject eye.
The solution of this object is achieved by the subject matter of the independent claim. The dependent claims contain advantageous embodiments of the present invention.

**In** order to solve the above-described problem, aspects of the present invention include the following configurations.
(1) A subjective optometry apparatus for subjectively measuring optical characteristics of a subject eye, including:
   a light projecting optical system that has a visual target presenting portion for emitting a target light flux that presents a visual target to the subject eye in a first angle-of-view range, and guides the target light flux emitted from the visual target presenting portion to the subject eye; and
   a control portion that controls the light projecting optical system to project a visual target presented by the target light flux emitted from the visual target presenting portion onto the subject eye in a second angle-of-view range that exceeds the first angle-of-view range, in which a principal ray of the target light flux in a upstream optical path between the visual target presenting portion and a second optical member disposed to be the closest to the visual target presenting portion in an optical axis direction of an optical path of the light projecting optical system is parallel to an optical axis of the upstream optical path.
(2) The subjective optometry apparatus according to the above-described (1),
   in which the control portion drives a driving portion to move a first optical member in an optical path of the light projecting optical system so as to project the visual target onto the subject eye in the second angle-of-view range.
(3) The subjective optometry apparatus according to the above-described (2),
   in which the control portion drives the driving portion to insert the first optical member into the optical path or to remove the first optical member from the optical path so as to project the visual target onto the subject eye in the second angle-of-view range.
(4) The subjective optometry apparatus according to the above-described (2) or (3),
   in which the control portion drives the driving portion to move the first optical member in an optical axis direction of the optical path.
(5) The subjective optometry apparatus according to any one of the above-described (1) to (4), further including:
   a setting portion that sets either a first mode for projecting the visual target in the first angle-of-view range or a second mode for projecting the visual target in the second angle-of-view range,
   in which the control portion drives the driving portion based on a mode set by the setting portion.
(6) The subjective optometry apparatus according to any one of the above-described (1) to (5),
   in which the control portion changes a projection magnification of the light projecting optical system to project the visual target onto the subject eye in the second angle-of-view range.
(7) The subjective optometry apparatus according to any one of the above-described (1) to (6), further including:
   a calibration optical system that is disposed in an optical path of the light projecting optical system and changes optical characteristics of the target light flux; and
   a subjective measurement portion that subjectively measures the optical characteristics of the subject eye.

### BRIEF DESCRIPTION OF DRAWINGS

Fig. 1 is an exterior view of an example of a subjective optometry apparatus.
Fig. 2 is a view illustrating a configuration of a measurement portion.
Fig. 3 is a schematic configuration view when the inside of the subjective optometry apparatus is seen from the front.
Fig. 4 is a schematic configuration view when the inside of the subjective optometry apparatus is seen from the side.
Fig. 5 is a schematic configuration view when the inside of the subjective optometry apparatus is seen from above.
Fig. 6 is a view illustrating a control system of the subjective optometry apparatus.
Fig. 7 is a view for describing an angle of view of a display and an angle of view of an examination visual target.
Figs. 8A to 8D are views illustrating a change in angle of view due to a change in projection magnification.
Figs. 9A and 9B are views for describing a change in the **number** of pixels due to a change in projection magnification.
Figs. 10A and 10B are views for describing switching of a projection lens that corresponds to a mode.
Figs. 11A and 11B are views for describing insertion and removal of a lens that corresponds to the mode.
Figs. 12 A and 12B are views for describing a case where a light projecting optical system is used as a relay optical system.
Figs. 13 A and 13B are views for describing insertion and removal of a mirror that corresponds to the mode.
Figs. 14 A and 14B are views for describing a comparative case where the principal ray of a target light flux is not parallel to an optical axis.
Figs. 15 A and 15B are views for describing a change in appearance of the examination visual target due to the switching of the mode.

### DETAILED DESCRIPTION

### <Outline>

A subjective optometry apparatus according to embodiments of the present disclosure will be described. Hereinafter, a description will be given on the assumption that a depth direction (a front-back direction of an examinee) of the subjective optometry apparatus is a Z direction, a horizontal direction (a left-right direction of the examinee) on a plane which is perpendicular to the depth direction is an X direction, and a vertical direction (an up-down direction of the examinee) on a plane which is perpendicular to the depth direction is a Y direction. In addition, hereinafter, L and R attached to the reference numerals indicate a left subject eye (left eye) and a right subject eye (right eye), respectively. Meanwhile, items classified as the following sign "< >" may be used independently of or in relation to each other.

For example, the subjective optometry apparatus (for example, a subjective optometry apparatus 1) according to the present embodiment may be a subjective optometry apparatus for subjectively measuring optical characteristics of a subject eye. Such a subjective optometry apparatus may be a visual target presenting apparatus capable of presenting a visual target (hereinafter, referred to as an examination visual target) to the subject eye at a predetermined examination distance. **In** addition, such a subjective optometry apparatus may be a subjective optometry apparatus including a subjective measurement portion for subjectively measuring the optical characteristics of the subject eye. **In** addition, in the present embodiment, as the subjective optometry apparatus, a subjective optometry apparatus including a subjective measurement portion is employed as an example.

**In** addition, the subjective optometry apparatus may include an objective measurement portion for objectively measuring the optical characteristics of the subject eye. It is needless to say that the subjective optometry apparatus may include both the subjective measurement portion and the objective measurement portion. Examples of the optical characteristics of the subject eye may include at least any one of an eye refractive power (for example, at least any one of a spherical power, a cylinder power, and an astigmatic axis angle), a contrast sensitivity, a binocular vision function (for example, at least any one of the amount of oblique position and a stereoscopic function), and the like.

The subjective optometry apparatus includes a light projecting optical system (for example, a light projecting optical system 30). The light projecting optical system projects a target light flux emitted from the visual target presenting portion (for example, display 31) toward the subject eye. **In** addition, for example, the subjective optometry apparatus may include a calibration optical system (for example, a calibration optical system 60 and a subjective measurement optical system 25). For example, the calibration optical system is disposed in an optical path of the light projecting optical system and changes the optical characteristics of the target light flux. **In** addition, for example, the subjective optometry apparatus may include an optical member (for example, a concave surface mirror 85). For example, the optical member guides the target light flux calibrated by the calibration optical system to the subject eye.

For example, in the subjective optometry apparatus, the target light flux may be guided to the subject eye through an optical path that matches an optical axis of an optical member. **In** other words, the subjective measurement portion may be configured to subjectively measure the optical characteristics of the subject eye by guiding the target light flux through the optical path that matches the optical axis of the optical member to the subject eye. **In** addition, for example, in the subjective optometry apparatus 1, the target light flux may be guided to the subject eye through a path deviated from the optical axis of the optical member. **In** other words, the subjective measurement portion may be configured to subjectively measure the optical characteristics of the subject eye by guiding the target light flux through the path deviated from the optical axis of the optical member to the subject eye.

### <Light Projecting Optical System>

The light projecting optical system includes a visual target presenting portion for emitting a target light flux which presents a visual target to the subject eye in a first angle-of-view range. Further, the light projecting optical system guides the target light flux emitted from the visual target presenting portion to the subject eye. For example, a configuration may also be adopted in which a display is used as the visual target presenting portion for emitting the target light flux. For example, as the display, a liquid crystal on silicon (LCOS), a liquid crystal display (LCD), an organic electro luminescence (EL), or the like is used. For example, the examination visual target, such as a landscape visual target or a Landolt ring visual target, is displayed on the display. In addition, for example, as the visual target presenting portion for emitting the target light flux, a light source and a digital micromirror device (DMD) may be used. In general, since the DMD has high reflectivity and brightness, it is possible to maintain a quantity of light of the target light flux compared to a case where a liquid crystal display using polarization is used. In addition, for example, as the visual target presenting portion for emitting the target light flux, a configuration including a visual target presenting visible light source and a visual target plate may be employed. For example, the visual target plate is a rotatable disk plate, and includes a plurality of examination visual targets. For example, the plurality of examination visual targets include a visual target for examination of visual acuity which is used during the subjective measurement, and the like. For example, as the visual target for examination of visual acuity, a visual target (visual acuity value 0.1, 0.3, ..., 1.5) is provided for each visual acuity value. For example, the visual target plate is rotated by a motor or the like, and the examination visual targets are disposed in a switching manner in an optical path through which the target light flux is guided to the subject eye. It is needless to say that the visual target presenting portion for emitting the target light flux may be other than the above-described configuration.

For example, the light projecting optical system may include a right eye light projecting optical system and a left eye light projecting optical system which are provided as a pair on the left and right sides respectively. For example, the left eye projecting optical system and the right eye projecting optical system may be configured such that members configuring the left eye projecting optical system and members configuring the right eye projecting optical system are the same members. In addition, for example, the left eye light projecting optical system and the right eye light projecting optical system may be configured such that at least some of the members configuring the left eye light projecting optical system and the members configuring the right eye light projecting optical system are members different from each other. In addition, for example, the left eye light projecting optical system and the right eye light projecting optical system may be configured such that at least some of the members configuring the left eye light projecting optical system and the members configuring the right eye light projecting optical system are used in common. In addition, for example, the left eye light projecting optical system and the right eye light projecting optical system may be configured such that the members configuring the left eye light projecting optical system and the members configuring the right eye light projecting optical system are separately provided.

### <Calibration Optical System>

For example, the calibration optical system may be configured to change optical characteristics (for example, at least any one of a spherical power, a cylindrical power, astigmatism information, polarization characteristics, and an optical aberration) of the target light flux. For example, as a configuration in which the optical characteristics of the target light flux is changed, a configuration in which an optical element is controlled may be adopted. For example, as the optical element, a configuration may also be adopted in which at least any one of a spherical lens, a cylindrical lens, a cross cylinder lens, a rotary prism, a wavefront modulation element, and the like is used. Naturally, for example, as the optical element, an optical element different from the optical element having the above-described configuration may be used.

For example, the calibration optical system may be configured such that a spherical power of the subject eye is calibrated by a presentation position (presenting distance) of the examination visual target with respect to the subject eye is optically changed. In this case, for example, as a configuration in which the presentation position (presenting distance) of the examination visual target is optically changed, a configuration may also be adopted in which a light source (for example, a display) is moved in an optical axis direction. Further, for example, as a configuration in which the presentation position (presenting distance) of the examination visual target is optically changed, a configuration may also be adopted in which the optical element (for example, the spherical lens) disposed in the optical path is moved in the optical axis direction. Naturally, the calibration optical system may have a configuration constituted by a configuration in which the optical element is controlled and a configuration in which the optical element disposed in the optical path is moved in the optical axis direction.

For example, the calibration optical system may be an optometry unit (phoropter) in which optical elements disposed in front of the subject eye are disposed in a switching manner. For example, the optometry unit may be configured to include a lens disc having a plurality of optical elements disposed on the same circumference thereof and a driving portion for rotating the lens disc, and to electrically switch the optical elements by the driving of the driving portion (for example, a motor).

For example, the calibration optical system may be configured to change the optical characteristics of the target light flux by disposing the optical element between the optical member for guiding the target light flux toward the subject eye from the light projecting optical system and the light source of the light projecting optical system and by controlling the optical element. In other words, the calibration portion may have a configuration of a phantom lens refractometer (phantom calibration optical system). In this case, for example, the target light flux calibrated by the calibration optical system is guided to the subject eye through the optical member.

For example, the calibration optical system may include a left eye calibration optical system and a right eye calibration optical system which are provided as a pair on the left and right sides respectively. For example, the left eye calibration optical system and the right eye calibration optical system may be configured such that members configuring the left eye calibration optical system and members configuring the right eye calibration optical system are the same members. In addition, for example, the left eye calibration optical system and the right eye calibration optical system may be configured such that at least some of the members configuring the left eye calibration optical system and the members configuring the right eye calibration optical system are members different from each other. In addition, for example, the left eye calibration optical system and the right eye calibration optical system may be configured such that at least some of the members configuring the left eye calibration optical system and the members configuring the right eye calibration optical system are used in common. **In** addition, for example, the left eye calibration optical system and the right eye calibration optical system may be configured such that the members configuring the left eye calibration optical system and the members configuring the right eye calibration optical system are separately provided.

### <Control Portion>

The subjective optometry apparatus in the present embodiment includes a control portion (for example, a control portion 70). The control portion controls the light projecting optical system to project an examination visual target presented by the target light flux emitted from the visual target presenting portion to the subject eye in a second angle-of-view range that exceeds the first angle-of-view range that can be presented by the target light flux emitted from the visual target presenting portion. **In** other words, the control portion projects the examination visual target of an angle of view in the second angle-of-view range onto the subject eye by controlling the light projecting optical system. The second angle-of-view range is an angle-of-view range that exceeds at least any one of an angle-of-view range greater (wider) than the first angle-of-view range and an angle-of-view range smaller (narrower) than first angle-of-view range. **In** other words, the second angle-of-view range that exceeds the first angle-of-view range is an angle-of-view range greater than the first angle-of-view range. **In** addition, for example, the second angle-of-view range that exceeds the first angle-of-view range may be an angle-of-view range smaller than the first angle-of-view range. It is needless to say that the second angle-of-view range that exceeds the first angle-of-view range may include both a range of an angle of view greater than the first angle-of-view range and a range of the angle of view smaller than the first angle-of-view range. According to this, it is possible to further widen the range in which the angle of view of the examination visual target projected onto the subject eye can be changed. In other words, it is possible to project the examination visual targets having various angles of view onto the subject eye.

For example, the control portion drives the driving portion (for example, a driving mechanism 43) to move the first optical member (for example, a projection lens 33, a projection lens 40, and the like) in the optical path of the light projecting optical system so as to project the examination visual target onto the subject eye in the second angle-of-view range. For example, in the present embodiment, the first optical member may be configured with at least one optical member. For example, the first optical member may be at least any one of a lens, a prism, a mirror, and the like. Further, for example, the first optical member may be any optical member of the light projecting optical system, or may be an optical member provided separately from the optical member of the light projecting optical system.

For example, the movement of the first optical member may be configured to move the first optical member in the optical axis direction of the optical path. In other words, the control portion may drive the driving portion to move the first optical member in the optical axis direction of the optical path so as to project the examination visual target onto the subject eye in the second angle-of-view range. In this case, the first optical member may be moved parallel to the optical axis. In addition, in this case, the first optical member may be moved in a diagonal direction to the optical axis. In addition, for example, the movement of the first optical member may be configured to move the first optical member in a direction different from the optical axis direction of the optical path. In this case, the first optical member may be moved perpendicular to the optical axis.

In addition, for example, the movement of the first optical member may be configured to insert the first optical member to the optical path or to remove the first optical member from the optical path. In other words, the control portion may drive the driving portion to insert and remove the first optical member to and from the optical path so as to project the examination visual target onto the subject eye in the second angle-of-view range. In this case, at least any one of a configuration in which the first optical member is inserted into the optical path, a configuration in which the first optical member is removed from the optical path, a configuration in which the first optical member is switched, or the like may be used.

For example, in this manner, the control portion drives the driving portion to move the first optical member in the optical path of the light projecting optical system, and accordingly, it is possible to change the angle-of-view range of the examination visual target projected onto the subject eye without providing a complicated configuration.

For example, the control portion may change the projection magnification of the light projecting optical system to project the examination visual target onto the subject eye in the first angle-of-view range. In addition, for example, the control portion may change the projection magnification of the light projecting optical system to project the examination visual target onto the subject eye in the second angle-of-view range. For example, the projection magnification of the light projecting optical system may be changed by changing the size of the examination visual target presented by the visual target presenting portion. In addition, for example, the projection magnification of the light projecting optical system may be changed by moving the optical member. In this case, the optical member that can move in the optical path of the light projecting optical system and the driving portion for moving the optical member in the optical path of the light projecting optical system may be provided. In addition, such an optical member may be a lens, a prism, a mirror, or the like. In addition, such an optical member may be used as the first optical member, or may be provided separately from the first optical member.

### <Setting Portion>

For example, the subjective optometry apparatus in the present embodiment may include a setting portion (for example, a control portion 70). For example, the setting portion sets either a first mode of projecting the examination visual target in the first angle-of-view range or a second mode of projecting the examination visual target in the second angle-of-view range. For example, the modes may be set based on the angle-of-view information. For example, the angle-of-view information may be information that can acquire the angle of view of the examination visual target projected onto the subject eye. In this case, the angle-of-view information may be the angle of view itself of the examination visual target projected onto the subject eye. In addition, in this case, the angle-of-view information may be the size of the examination visual target projected onto the subject eye. It is needless to say that the angle-of-view information may be the size of the examination visual target presented by the visual target presenting portion. For example, in the present embodiment, such angle-of-view information is acquired from the visual acuity value of the examination visual target projected onto the subject eye.

For example, the setting portion may automatically set either the first mode or the second mode based on the angle-of-view information. It is needless to say that either the first mode or the second mode may be manually set by an examiner. In this case, a configuration in which the setting of the mode can be switched by an operation portion (for example, a monitor 4) provided in the subjective optometry apparatus may be employed. For example, the control portion drives the driving portion based on the mode set by the setting portion. Accordingly, the first optical member is moved in accordance with each mode, and the examination visual target in the first angle-of-view range or the second angle-of-view range can be projected onto the subject eye.

In addition, the mode set in the present embodiment is not limited to the first mode and the second mode. For example, a configuration in which a mode that can be set is provided in addition to the modes may be employed. In other words, in the present embodiment, a plurality of modes in which the examination visual targets having different angle-of-view ranges can be projected onto the subject eye may be settable. In this case, for example, a configuration in which a mode for projecting the examination visual target in a greater angle-of-view range, a mode for projecting the examination visual target in a smaller angle-of-view range, and a mode for projecting the examination visual target in moderate angle-of-view range are provided may be employed.

The principal ray of the target light flux in a upstream optical path between the visual target presenting portion and the second optical member disposed to be the closest to the visual target presenting portion in the optical axis direction of the optical path of the light projecting optical system is parallel to an optical axis of the upstream optical path. For example, the second optical member may be used as the first optical member, or may be an optical member different from the first optical member. For example, the second optical member may be an optical member fixedly disposed to be the closest to the visual target presenting portion in the optical axis direction. **In** addition, for example, the second optical member may be an optical member disposed to be the closest to the visual target presenting portion in the optical axis direction and to be movable. Accordingly, even when the eye refractive power of the subject eye changes, the examination visual target having the same size can be projected onto the subject eye. **In** addition, it is possible to suppress occurrence of color unevenness or the like in the examination visual target.

**In** a comparative example, the principal ray of the target light flux in the upstream optical path between the visual target presenting portion and the second optical member disposed to be the closest to the visual target presenting portion in the optical axis direction is not be parallel to the optical axis of the upstream optical path. **In** this case, when the eye refractive power of the subject eye changes, the examination visual target having the same size cannot be projected onto the subject eye. **In** this case, the correction processing for projecting the examination visual target having the same size onto the subject eye may be performed regardless of the eye refractive power of the subject eye. **In** this example, the correction processing may be performed in which the size of the examination visual target presented by the visual target presenting portion is changed in advance in accordance with the eye refractive power of the subject eye.

**In** addition, the present disclosure is not limited to the apparatus described in the present embodiment. For example, terminal control software (program) for performing functions of the embodiments which will be described below is supplied to a system or an apparatus via a network or various storage media, and a control device (for example, a CPU or the like) of the system or the apparatus can also read the program.

### <Example>

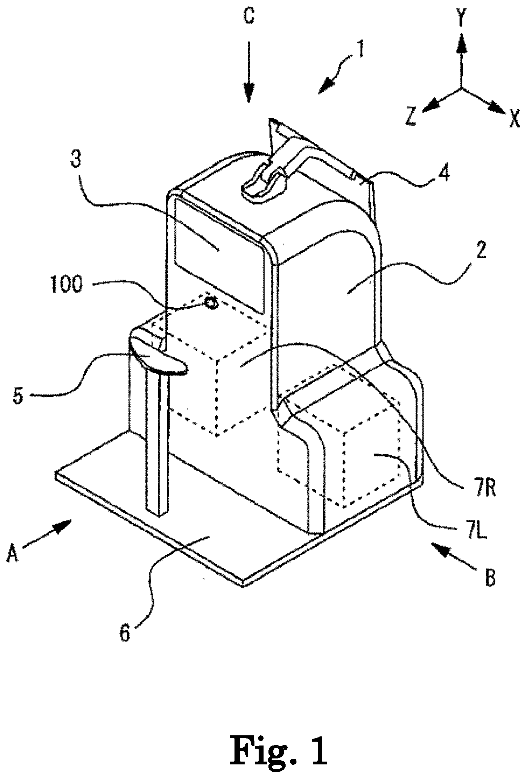

In the example of the present disclosure, the description will be made with reference to Figs. 1 to 15. Fig. 1 illustrates an exterior view of the subjective optometry apparatus 1 according to the present example. For example, the subjective optometry apparatus 1 includes a housing 2, a presentation window 3, a monitor (display) 4, a chin mount 5, a base 6, an anterior ocular segment image capture optical system 100, and the like. For example, the housing 2 includes a measurement portion 7 on the inside thereof (details thereof will be described later). For example, the presentation window 3 is used to present the examination visual target to examinee. For example, the target light flux from the measurement portion 7 is projected onto a subject eye E of the examinee via the presentation window 3.

For example, the monitor 4 displays the optical characteristics result (for example, spherical power, cylindrical power, astigmatic axis angle, and the like) of the subject eye E. For example, the monitor 4 is a touch panel. In other words, in the present example, the monitor 4 functions as an operation portion (controller). For example, the signal that corresponds to an operation instruction input from the monitor 4 is output to the control portion 70 which will be described later. In addition, the monitor 4 may not be a touch panel type, or may be configured to separately provide the monitor 4 and the operation portion. For example, in this case, the operation portion may be configured to use at least one operation portion, such as a mouse, a joystick, or a keyboard.

For example, the monitor 4 may be a monitor mounted on the housing 2, or may be a monitor connected to the housing 2. For example, in this case, a configuration using the monitor of a personal computer may be used. In addition, for example, a plurality of monitors may be used together.

For example, the distance between the subject eye E and the subjective optometry apparatus 1 is kept constant by the chin mount 5. In the present example, the configuration using the chin mount 5 is used as an example to keep the distance between the subject eye E and the subjective optometry apparatus 1 constant, but the invention is not limited thereto. For example, in the present example, a configuration may be adopted in which a forehead protector, a face protector, and the like are used in order to keep the distance between the subject eye E and the subjective optometry apparatus 1 constant. For example, the chin mount 5 and the housing 2 are fixed to the base 6.

For example, the anterior ocular segment image capture optical system 100 is configured with an image capture element and a lens which are not illustrated in the drawing. For example, the anterior ocular segment image capture optical system 100 is used to capture an image of the face of the examinee.

### <Measurement Portion>

For example, the measurement portion 7 includes a left eye measurement portion 7L and a right eye measurement portion 7R. In other words, the subjective optometry apparatus 1 in the present example includes a pair of left and right subjective measurement portions and a pair of left and right objective measurement portions. For example, the left eye measurement portion 7L and the right eye measurement portion 7R in the present example include the same member. Naturally, the left eye measurement portion 7L and the right eye measurement portion 7R may be configured such that at least some members thereof are different from each other.

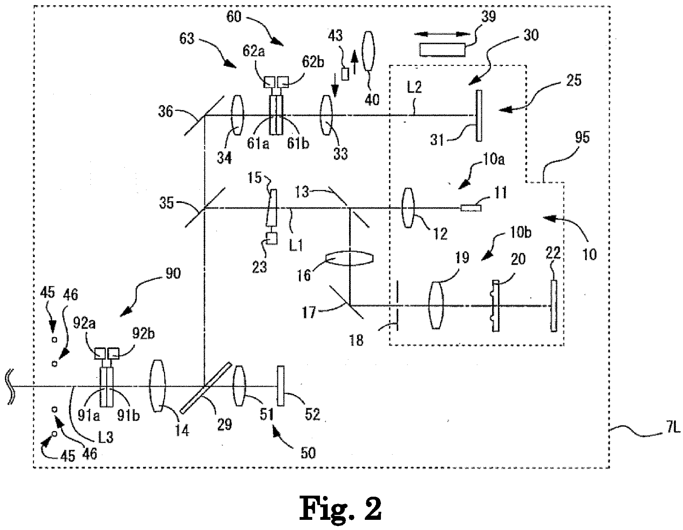

Fig. 2 is a view illustrating a configuration of the measurement portion 7. For example, in the present example, the left eye measurement portion 7L is described as an example. In addition, since the right eye measurement portion 7R has the same configuration as that of the left eye measurement portion 7L, the description thereof will be omitted. For example, the left eye measurement portion 7L includes the subjective measurement optical system 25, the objective measurement optical system 10, the first index projection optical system 45, the second index projection optical system 46, and the observation optical system 50.

### <Subjective Optical System>

For example, the subjective measurement optical system 25 is used as a part of the configuration of the subjective measurement portion for subjectively measuring optical characteristics of the subject eye E (details thereof will be described later). Examples of the optical characteristics of the subject eye E include an eye refractive power, a contrast sensitivity, a binocular vision function (for example, the amount of oblique position, a stereoscopic function, and the like), and the like. In addition, in the present example, an example of the subjective measurement portion for measuring the eye refractive power of the subject eye E will be described. For example, the subjective measurement optical system 25 includes the light projecting optical system (visual target projection system) 30, the calibration optical system 60, and a correction optical system 90.

For example, the light projecting optical system 30 projects the target light flux toward the subject eye E. For example, the light projecting optical system 30 includes the display 31, the projection lens 40, the projection lens 33, a projection lens 34, a reflecting mirror 36, a dichroic mirror 35, a dichroic mirror 29, and an objective lens 14. For example, in a first mode which will be described later, the target light flux emitted from the display 31 is projected onto the subject eye E through the optical member in order of the projection lens 33, the projection lens 34, the reflecting mirror 36, the dichroic mirror 35, the dichroic mirror 29, and the objective lens 14. In addition, for example, in a second mode which will be described later, the target light flux emitted from the display 31 is projected to the subject eye E via optical members in the order of projection lens 40, the projection lens 33, the projection lens 34, the reflecting mirror 36, the dichroic mirror 35, the dichroic mirror 29, and the objective lens 14.

For example, an examination visual target, such as a Landolt ring visual target, a fixation target for fixedly viewing the subject eye E, and the like are displayed on the display 31. For example, the target light flux emitted from the display 31 is projected toward the subject eye E. For example, in the present example, as the display 31, the LCOS is used. In addition, as a display, at least any one of an LCD, an organic EL display, a plasma display, or the like can also be used.

For example, the driving mechanism 43 (for example, a motor, a slide mechanism, and the like) for moving the lenses is provided in the projection lens 33 and the projection lens 40. For example, the projection lens 33 is moved by the driving mechanism 43. In addition, for example, the projection lens 40 is moved by the driving mechanism 43. In addition, the driving mechanism 43 is configured with a driving mechanism for moving the projection lens 33 and a driving mechanism for moving the projection lens 40, and the respective lenses may be moved separately. For example, in the present example, by driving the driving mechanism 43, the projection lens 33 and the projection lens 40 are inserted into the optical path of the light projecting optical system 30 or removed from the optical path. For example, in the first mode which will be described later, the projection lens 33 is disposed in the optical path and the projection lens 40 is disposed out of the optical path. In addition, in the second mode which will be described later, the projection lens 33 is disposed out of the optical path and the projection lens 40 is disposed in the optical path. It is needless to say that both the projection lens 33 and the projection lens 40 may be disposed in the optical path and be appropriately inserted and removed in accordance with the set mode. In addition, both the projection lens 33 and the projection lens 40 may be disposed out of the optical path and be appropriately inserted in accordance with the set mode.

For example, the calibration optical system 60 is disposed in the optical path of the light projecting optical system 30. For example, the calibration optical system 60 changes the optical characteristics of the target light flux. For example, the calibration optical system 60 includes an astigmatism calibration optical system 63 and a driving mechanism 39. For example, the astigmatism calibration optical system 63 is disposed between the projection lens 33 and the projection lens 34. For example, the astigmatism calibration optical system 63 is used for calibrating a cylindrical power and an astigmatic axis angle of the subject eye E. For example, the astigmatism calibration optical system 63 is configured with two positive cylindrical lenses 61a and 61b having the same focal length. The cylindrical lenses 61a and 61b are independently rotated around an optical axis L2 by the driving of respective rotation mechanisms 62a and 62b. Meanwhile, in the present example, the astigmatism calibration optical system 63 has been described using an example of a configuration in which the two positive cylindrical lenses 61a and 61b are used, but the invention is not limited thereto. The astigmatism calibration optical system 63 may be configured to be capable of calibrating a cylindrical power, an astigmatic axis angle, and the like. In this case, a configuration may also be adopted in which the calibration lens is inserted into and removed from the optical path of the light projecting optical system 30.

For example, the driving mechanism 39 is configured with a motor and a slide mechanism. For example, by the driving mechanism 39, the display 31 is integrally moved in the optical axis L2 direction. For example, the presentation position (presenting distance) of the examination visual target with respect to the subject eye E is optically changed by the movement of the display 31 during the subjective measurement, and a spherical refractive power of the subject eye E is calibrated. In other words, the calibration optical system of the spherical power is configured by the movement of the display 31. In addition, for example, fogging is applied to the subject eye E by the movement of the display 31 during the objective measurement. Meanwhile, the calibration optical system of the spherical power is not limited thereto. For example, the calibration optical system of the spherical power includes a large number of optical elements, and may be configured to perform calibration by the optical elements being disposed in the optical path. In addition, for example, the calibration optical system of the spherical power may be configured to move the lens disposed in the optical path in the optical axis direction.

Meanwhile, in the present example, an example of the calibration optical system for calibrating a spherical power, a cylindrical power, and an astigmatic axis angle has been described, but the invention is not limited thereto. For example, the calibration optical system for calibrating a prism value may be provided. The calibration optical system for the prism value is provided, and thus, it is possible to perform calibration such that the target light flux is projected onto the subject eye E even when the examinee has heterophoria.

Meanwhile, in the present example, a description has been given of an example of a configuration in which the astigmatism calibration optical system 63 for calibrating the cylindrical power and the astigmatic axis angle and the calibration optical system (for example, the driving mechanism 39) for calibrating the spherical power are separately provided, but the invention is not limited thereto. For example, as the calibration optical system, a configuration may be adopted in which a calibration optical system for calibrating a spherical power, a cylindrical power, and an astigmatic axis angle is provided. In other words, the calibration optical system in the present example may be an optical system for modulating the wavefront. In addition, for example, the calibration optical system may be an optical system that calibrates a spherical power, a cylindrical power, an astigmatic axis angle, and the like. In this case, for example, the calibration optical system may be configured to include a lens disc on which a large number of optical elements (a spherical lens, a cylindrical lens, a dispersing prism, and the like) are disposed on the same circumference. The lens disc is rotationally controlled by the driving portion (an actuator, a stepping motor, and the like), and the optical element (for example, a cylindrical lens, a cross cylinder lens, a rotary prism, and the like) desired by the examiner is disposed on the optical axis L2 at the rotation angle desired by the examiner. For example, the switching of the optical element disposed on the optical axis L2, and the like may be performed by the operation of the monitor 4 or the like.

The lens disc is configured with one lens disc or a plurality of lens discs. In a case where a plurality of lens discs are disposed, the driving portion that corresponds to each of the lens discs is provided. For example, as a lens disc group, each of the lens discs has an opening (or a 0D lens) and a plurality of optical elements. As a type of each of the lens discs, a spherical lens disc having a plurality of spherical lenses with different frequencies, a cylindrical lens disc having a plurality of cylindrical lenses with different frequencies, and an auxiliary lens disc having a plurality of types of auxiliary lenses are representative. At least one of a red filter and a green filter, a prism, a cross cylinder lens, a polarizing plate, a Maddox lens, and an autocross cylinder lens is disposed on the auxiliary lens disc. In addition, the cylindrical lens is rotatably disposed around the optical axis L2 by the driving portion, and the rotary prism and the cross cylinder lens may be disposed to be rotatable around each of the optical axes by the driving portion.

For example, the correction optical system 90 is disposed between the objective lens 14 and a deflection mirror 81 which will be described later. For example, the correction optical system 90 is used for correcting optical aberrations (for example, astigmatism) generated in the subjective measurement. For example, the correction optical system 90 is configured with two positive cylindrical lenses 91a and 91b having the same focal length. For example, the correction optical system 90 corrects the astigmatism by adjusting the cylindrical power and the astigmatic axis angle. Each of the cylindrical lens 91a and the cylindrical lens 91b is independently rotated around an optical axis L3 by driving the rotation mechanisms 92a and 92b, respectively. In addition, in the present example, the configuration using two positive cylindrical lenses 91a and 91b has been described as an example of the correction optical system 90, but the present invention is not limited thereto. The correction optical system 90 may have any configuration as long as the configuration can correct the astigmatism. In this case, for example, the correction lens may be inserted into and removed from the optical axis L3.

In addition, in the present example, the configuration in which the correction optical system 90 is disposed separately from the calibration optical system 60 has been described as an example, but the invention is not limited thereto. For example, the calibration optical system 60 may be configured to also serve as the correction optical system 90. In this case, the cylindrical power of the subject eye E and the astigmatic axis angle are corrected in accordance with the amount of astigmatism. In other words, the calibration optical system 60 is driven so as to correct the cylindrical power considering (correcting) the astigmatism amount or the astigmatic axis angle. For example, by using both the calibration optical system 60 and the correction optical system 90, complicated control is not required, and thus, it is possible to correct the optical aberration with a simple configuration. In addition, for example, by using both the calibration optical system 60 and the correction optical system 90, it is not necessary to separately provide the correction optical system for the optical aberration, and thus, it is possible to correct the optical aberration with a simple configuration.

### <Objective Optical System>

For example, the objective measurement optical system 10 is used as a part of a configuration of the objective measurement portion for objectively measuring the optical characteristics of the subject eye (details thereof will be described later). Examples of the optical characteristics of the subject eye E include an eye refractive power, an ocular axial length, a cornea shape, and the like. In the example, an example of the objective measurement portion for measuring an eye refractive power of the subject eye E will be described. For example, the objective measurement optical system 10 includes a projection optical system 10a, a light receiving optical system 10b, and a correction optical system 90.

For example, the projection optical system (light projecting optical system) 10a projects a spot-shaped measurement index onto a fundus of the subject eye E through the pupil center portion of the subject eye E. For example, the light receiving optical system 10b extracts fundus reflected light reflected from the fundus in a ring shape through a pupil peripheral portion, and causes an image capture element 22 to capture a ring-shaped fundus reflected image.

For example, the projection optical system 10a includes a light source 11, a relay lens 12, a hole mirror 13, a prism 15, a driving portion (motor) 23, a dichroic mirror 35, a dichroic mirror 29, and an objective lens 14 which are disposed on an optical axis L1 of the objective measurement optical system 10. For example, the prism 15 is a luminous flux deflection member. For example, the driving portion 23 rotationally drives the prism 15 around the optical axis L1. For example, the light source 11 has a conjugate relationship with the fundus of the subject eye E. Further, the hole portion of the hole mirror 13 has a conjugate relationship with the pupil of the subject eye E. For example, the prism 15 is disposed at a position away from the position conjugated with the pupil of the subject eye E, and the luminous flux to pass through the prism is eccentric with the optical axis L1. Meanwhile, a configuration may also be adopted in which a parallel plane plate is obliquely disposed on the optical axis L1 as the luminous flux deflection member instead of the prism 15.

For example, the dichroic mirror 35 is common to the optical path of the subjective measurement optical system 25 and the optical path of the objective measurement optical system 10. In other words, for example, the dichroic mirror 35 has the optical axis L2 of the subjective measurement optical system 25 and the optical axis L1 of the objective measurement optical system 10 as the same axis. For example, the dichroic mirror 29 which is an optical path branching member reflects the luminous flux of the subjective measurement optical system 25 and the measurement light of the projection optical system 10a, and guides the reflected luminous flux and measurement light to the subject eye E.

For example, the light receiving optical system 10b uses the objective lens 14, the dichroic mirror 29, the dichroic mirror 35, the prism 15, and the hole mirror 13 in common with the projection optical system 10a, and includes a relay lens 16 disposed in the optical path in the reflection direction of the hole mirror 13, a mirror 17, a light receiving diaphragm 18 disposed in the optical path in the reflection direction of the mirror 17, a collimator lens 19, a ring lens 20, and the image capture element 22, such as a CCD. For example, the light receiving diaphragm 18 and the image capture element 22 has a conjugate relationship with the fundus of the subject eye E. For example, the ring lens 20 is configured with a lens portion formed in a ring shape and a light shielding portion obtained by performing coating for light shielding in a region other than the lens portion, and has an optically conjugate positional relationship with the pupil of the subject eye E. For example, an output from the image capture element 22 is input to the control portion 70.

For example, the dichroic mirror 29 reflects the reflected light of the measurement light from the projection optical system 10a guided to the fundus of the subject eye E toward the light receiving optical system 10b. In addition, for example, the dichroic mirror 29 transmits anterior ocular segment observation light and alignment light, and guides the transmitted light to the observation optical system 50. For example, the dichroic mirror 35 reflects the reflected light of the measurement light from the projection optical system 10a guided to the fundus of the subject eye E toward the light receiving optical system 10b.

Meanwhile, the objective measurement optical system 10 is not limited to the above-described objective measurement optical system, and it is possible to use a well-known objective measurement optical system configured to project a ring-shaped measurement index onto the fundus from the pupil peripheral portion, to extract the fundus reflected light from the pupil center portion, and to cause the image capture element 22 to receive light of the ring-shaped fundus reflected image.

Meanwhile, the objective measurement optical system 10 is not limited to the above-described objective measurement optical system, and may be a measurement optical system including a light projecting optical system which projects the measurement light toward the fundus of the subject eye E and a light receiving optical system in which the reflected light acquired by the reflection of the measurement light from the fundus is received by a light receiving element. For example, an eye refractive power measurement optical system may be configured to include a Shack Hartman sensor. Naturally, an apparatus using another measurement method may be used (for example, an apparatus of a phase difference system which projects a slit).

For example, the light source 11 of the projection optical system 10a, and the light receiving diaphragm 18, the collimator lens 19, the ring lens 20, and the image capture element 22 of the light receiving optical system 10b can be integrally moved in the optical axis direction. In the present example, for example, the light source 11 of the projection optical system 10a and the light receiving diaphragm 18, the collimator lens 19, the ring lens 20, and the image capture element 22 of the light receiving optical system 10b are integrally moved in the optical axis direction L1 by the driving mechanism 39 that drives the display 31. In other words, the display 31, the light source 11 of the projection optical system 10a, the light receiving diaphragm 18, the collimator lens 19, the ring lens 20, and the image capture element 22 of the light receiving optical system 10b are integrally moved as a driving unit 95 in synchronization with each other. Naturally, a configuration in which the components are separately driven may also be adopted.

For example, the driving unit 95 moves a part of the objective measurement optical system 10 in the optical axis direction such that an external ring luminous flux is incident on the image capture element 22 with respect to each longitudinal direction. In other words, a part of the objective measurement optical system 10 is moved in the optical axis direction L1 in accordance with a spherical refractive error (spherical refractive power) of the subject eye E, such that the spherical refractive error is corrected and the light source 11, the light receiving diaphragm 18, and the image capture element 22 are optically conjugated with the fundus of the subject eye E. For example, the position of the driving mechanism 39 to be moved is detected by a potentiometer not illustrated in the drawing. Meanwhile, the hole mirror 13 and the ring lens 20 are disposed so as to be conjugated with the pupil of the subject eye E with a fixed magnification, regardless of the amount of movement of the driving unit 95.

In the above-described configuration, measurement luminous flux emitted from the light source 11 forms a spot-shaped point light source image on the fundus of the subject eye E through the relay lens 12, the hole mirror 13, the prism 15, the dichroic mirror 35, the dichroic mirror 29, and the objective lens 14. At this time, a pupil projection image (projected luminous flux on the pupil) of the hole portion in the hole mirror 13 is eccentrically rotated at high speed by the prism 15 rotating around the optical axis. The point light source image projected onto the fundus is reflected and scattered, is emitted from the subject eye E, is condensed by the objective lens 14, and is condensed again at the position of the light receiving diaphragm 18 through the dichroic mirror 29, the dichroic mirror 35, the prism 15 which rotates at high speed, the hole mirror 13, the relay lens 16, and the mirror 17, thereby forming a ring-shaped image on the image capture element 22 by the collimator lens 19 and the ring lens 20.

For example, the prism 15 is disposed in an optical path which is common to the projection optical system 10a and the light receiving optical system 10b. For example, a reflected luminous flux from the fundus passes through the prism 15 which is the same as that of the projection optical system 10a, and thus, backward scanning is performed as if there is no eccentricity of the projected luminous flux and the reflected luminous flux (received luminous flux) on the pupil in the subsequent optical systems.

For example, the correction optical system 90 also serves as the subjective measurement optical system 25. Naturally, a configuration may also be adopted in which a correction optical system used in the objective measurement optical system 10 is separately provided.

### <First Index Projection Optical System and Second Index Projection Optical System>

For example, in the present example, the first index projection optical system 45 and the second index projection optical system 46 are disposed between the correction optical system 90 and the deflection mirror 81. Naturally, the arrangement position of the first index projection optical system 45 and the second index projection optical system 46 are not limited thereto. For example, the first index projection optical system 45 and the second index projection optical system 46 may be provided in a cover of the housing 2. For example, in this case, the first index projection optical system 45 and the second index projection optical system 46 are arranged around the presentation window 3.

For example, in the first index projection optical system 45, a plurality of infrared light sources are disposed on the concentric circle around the optical axis L3 at intervals of 45 degrees, and are disposed so as to be bilaterally symmetrical to each other with a vertical plane passing through the optical axis L3 therebetween. For example, the first index projection optical system 45 emits near infrared light for projecting an alignment index onto the cornea of the subject eye E. For example, the second index projection optical system 46 includes six infrared light sources which are disposed at a position different from the position of the first index projection optical system 45. In this case, the first index projection optical system 45 is configured to project an index at an infinite distance onto the cornea of the subject eye E from the left-right direction, and the second index projection optical system 46 is configured to project an index at a finite distance onto the cornea of the subject eye E from the up-down direction or an oblique direction. Meanwhile, in Fig. 2, only a part of the first index projection optical system 45 and the second index projection optical system 46 is illustrated for convenience of description. Meanwhile, the second index projection optical system 46 is also used as an anterior ocular segment illumination that illuminates the anterior ocular segment of the subject eye E. In addition, the second index projection optical system 46 can also be used as an index for measuring the shape of the cornea. In addition, the first index projection optical system 45 and the second index projection optical system 46 are not limited to a dot-shaped light source. For example, the systems may be a ring-shaped light source or a linear light source.

### <Observation Optical System>

For example, the observation optical system (image capture optical system) 50 shares the objective lens 14 and the dichroic mirror 29 in the subjective measurement optical system 25 and the objective measurement optical system 10, and includes an imaging lens 51 and an image capture element 52. For example, the image capture element 52 has an imaging surface disposed at a position substantially conjugated with the anterior ocular segment of the subject eye E. For example, an output from the image capture element 52 is input to the control portion 70. Accordingly, an anterior ocular segment image of the subject eye E is captured by the image capture element 52 and is displayed on the monitor 4. Meanwhile, the observation optical system 50 also serves as an optical system that detects an alignment index image formed on the cornea of the subject eye E by the first index projection optical system 45 and the second index projection optical system 46, and the position of the alignment index image is detected by the control portion 70.

### <Internal Configuration of Subjective Optometry Apparatus>

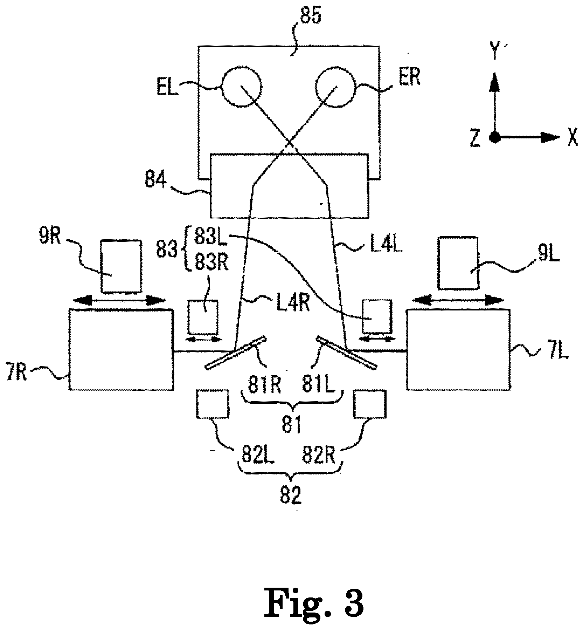

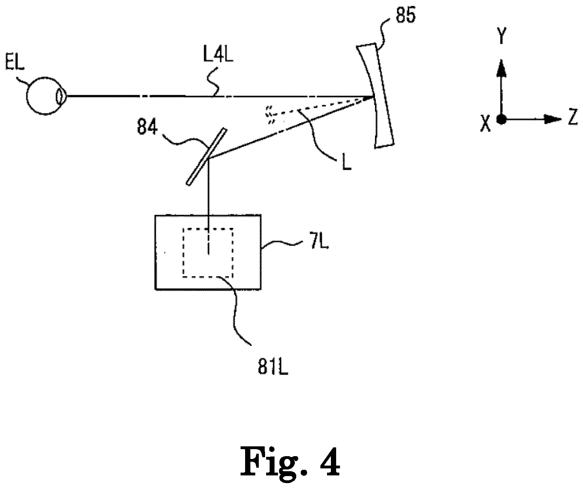

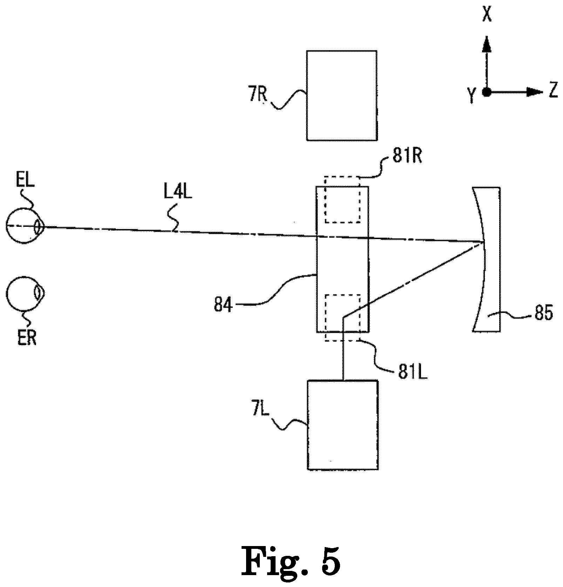

Hereinafter, the internal configuration of the subjective optometry apparatus 1 will be described. Fig. 3 is a schematic configuration view when the inside of the subjective optometry apparatus 1 according to the present example is seen from the front (a direction A of Fig. 1). Fig. 4 is a schematic configuration view when the inside of the subjective optometry apparatus 1 according to the present example is seen from the side (a direction B of Fig. 1). Fig. 5 is a schematic configuration view when the inside of the subjective optometry apparatus 1 according to the present example is seen from above (a direction C of Fig. 1). Meanwhile, in Figs. 4 and 5, only the optical axis of the left eye measurement portion 7L is illustrated for convenience of description.

For example, the subjective optometry apparatus 1 includes a subjective measurement portion and an objective measurement portion. For example, in the subjective measurement portion and the objective measurement portion, the target light flux from the measurement portion 7 passes through the optical path that matches an optical axis L of the optical member (for example, the concave surface mirror 85 which will be described later) and is guided to the subject eye E. In addition, for example, in the subjective measurement portion and the objective measurement portion, the target light flux from the measurement portion 7 may pass through the optical path deviated from the optical axis L of the optical member (for example, the concave surface mirror 85 which will be described later) and may be guided to the subject eye E. For example, the optical axis L in the present example is an axis toward the center of the sphere of the concave surface mirror 85. In addition, hereinafter, a configuration in which the target light flux from the measurement portion 7 passes through a path deviated from the optical axis L of the concave surface mirror 85 will be employed as an example. In other words, the target light flux from the measurement portion 7 is emitted obliquely to the optical axis L of the concave surface mirror 85, and the reflected luminous flux is guided to the subject eye E.

For example, the subjective measurement portion includes the measurement portion 7, the deflection mirror 81, the driving mechanism 82, a driving portion 83, a reflecting mirror 84, and the concave surface mirror 85. In addition, the subjective measurement portion is not limited to such a configuration. For example, the configuration without the reflecting mirror 84 may be adopted. In this case, the target light flux from the measurement portion 7 may be emitted obliquely to the optical axis L of the concave surface mirror 85 after passing through the deflection mirror 81. In addition, for example, the configuration with the half mirror may be adopted. In this case, the target light flux from the measurement portion 7 may be emitted obliquely to the optical axis L of the concave surface mirror 85 through the half mirror, and the reflected luminous flux may be guided to the subject eye E. In addition, in the present example, the concave surface mirror 85 is disposed, but a convex lens may be disposed instead of the concave surface mirror 85.

For example, the objective measurement portion includes the measurement portion 7, the deflection mirror 81, the reflecting mirror 84, and reflecting concave surface mirror 85. In addition, the objective measurement portion is not limited to such a configuration. For example, the configuration without the reflecting mirror 84 may be adopted. In this case, the target light flux from the measurement portion 7 may be emitted obliquely to the optical axis L of the concave surface mirror 85 after passing through the deflection mirror 81. In addition, for example, the configuration with the half mirror may be adopted. In this case, the target light flux from the measurement portion 7 may be emitted obliquely to the optical axis L of the concave surface mirror 85 through the half mirror, and the reflected luminous flux may be guided to the subject eye E. In addition, in the present example, the concave surface mirror 85 is disposed, but a convex lens may be disposed instead of the concave surface mirror 85.

For example, the subjective optometry apparatus 1 includes a left eye driving portion 9L and a right eye driving portion 9R, and the left eye measurement portion 7L and the right eye measurement portion 7R can be moved in the X direction respectively. For example, the left eye measurement portion 7L and the right eye measurement portion 7R are moved, and accordingly, a distance between the deflection mirror 81 and the measurement portion 7 is changed, and the presentation position of a target light flux in the Z direction is changed. Accordingly, it is possible to guide the target light flux calibrated by the calibration optical system 60 to the subject eye E and to adjust the measurement portion 7 in the Z direction such that the image of the target light flux calibrated by the calibration optical system 60 is formed on the fundus of the subject eye E.

For example, the deflection mirror 81 includes a right eye deflection mirror 81R and a left eye deflection mirror 81L which are provided as a pair on the left and right sides respectively. For example, the deflection mirror 81 is disposed between the calibration optical system 60 and the subject eye E. In other words, the calibration optical system 60 in the present example includes the right eye calibration optical system and the left eye calibration optical system which are provided as a pair on the left and right sides respectively, the left eye deflection mirror 81L is disposed between the left eye calibration optical system and a left subject eye EL, and the right eye deflection mirror 81R is disposed between the right eye calibration optical system and a right subject eye ER. For example, it is preferable that the deflection mirror 81 is disposed at a position conjugated with the pupil.

For example, the left eye deflection mirror 81L reflects a luminous flux projected from the left eye measurement portion 7L, and guides the luminous flux onto the left subject eye EL. In addition, for example, the left eye deflection mirror 81L reflects the reflected light reflected by the left subject eye EL, and guides the reflected light to the left eye measurement portion 7L. For example, the right eye deflection mirror 81R reflects the luminous flux projected from the right eye measurement portion 7R, and guides the luminous flux to the right subject eye ER. In addition, for example, the right eye deflection mirror 81R reflects the reflected light reflected by the right subject eye ER, and guides the reflected light to the right eye measurement portion 7R. Meanwhile, in the present example, a description has been given of an example of a configuration in which the deflection mirror 81 is used as a deflection member that reflects the luminous flux projected from the measurement portion 7 and guides the luminous flux to the subject eye E, but the invention is not limited thereto. As the deflection member, any deflection member that reflects the luminous flux projected from the measurement portion 7 and guides the luminous flux to the subject eye E may be used. Examples of the deflection member include a prism, a lens, or the like.

For example, a driving mechanism 82 is configured with a motor (driving portion) or the like. For example, the driving mechanism 82 includes a driving mechanism 82L for driving the left eye deflection mirror 81L and a driving mechanism 82R for driving the right eye deflection mirror 81R. For example, the deflection mirror 81 is rotated and moved by the driving of the driving mechanism 82. For example, the driving mechanism 82 rotates the deflection mirror 81 around a rotation axis in the horizontal direction (X direction) and a rotation axis in the vertical direction (Y direction). In other words, the driving mechanism 82 rotates the deflection mirror 81 in the XY directions. Meanwhile, the rotation of the deflection mirror 81 may be performed in either the horizontal direction or the vertical direction.

For example, the driving portion 83 is configured with a motor (driving portion) or the like. For example, the driving portion 83 includes a driving portion 83L for driving the left eye deflection mirror 81L and a driving portion 83R for driving the right eye deflection mirror 81R. For example, the deflection mirror 81 is moved in the X direction by the driving of the driving portion 83. For example, a distance between the left eye deflection mirror 81L and the right eye deflection mirror 81R is changed by the movement of the left eye deflection mirror 81L and the right eye deflection mirror 81R, and thus it is possible to change a distance between a left eye optical path and a right eye optical path in the X direction in accordance with the pupillary distance of the subject eye E.

Meanwhile, for example, a plurality of deflection mirrors 81 may be provided in each of the left eye optical path and the right eye optical path. Examples of the configuration include a configuration in which two deflection mirrors are provided in each of the left eye optical path and the right eye optical path (for example, two deflection mirrors in the left eye optical path, or the like). In this case, one deflection mirror may be rotated in the X direction, and the other deflection mirror may be rotated in the Y direction. For example, the deflection mirror 81 is rotated and moved, and thus it is possible to optically correct the position of an image to be formed by deflecting an apparent luminous flux for the image of the calibration optical system 60 to be formed in front of the subject eye.

For example, the concave surface mirror 85 is shared by the right eye measurement portion 7R and the left eye measurement portion 7L. For example, the concave surface mirror 85 is shared by the right eye optical path including the right eye calibration optical system and the left eye optical path including the left eye calibration optical system. In other words, the concave surface mirror 85 is disposed at a position where the concave surface mirror passes through both the right eye optical path including the right eye calibration optical system and the left eye optical path including the left eye calibration optical system. Naturally, the concave surface mirror 85 may be configured not to be shared by the right eye optical path and the left eye optical path. In other words, a configuration may also be adopted in which the concave surface mirrors are provided in each of the right eye optical path including the right eye calibration optical system and the left eye optical path including the left eye calibration optical system. For example, the concave surface mirror 85 guides the target light flux having passed through the calibration optical system to the subject eye E, and forms an image of the target light flux having passed through the calibration optical system in front of the subject eye E. In addition, in the present embodiment, the configuration using the concave surface mirror 85 has been described as an example, but the invention is not limited thereto, and various optical members can be used. For example, as the optical member, a lens, a planar mirror, and the like can be used.

For example, the concave surface mirror 85 also serves as the subjective measurement portion and the objective measurement portion. For example, the target light flux projected from the subjective measurement optical system 25 is projected onto the subject eye E through the concave surface mirror 85. For example, the measurement light projected from the objective measurement optical system 10 is projected onto the subject eye E through the concave surface mirror 85. In addition, for example, the reflected light of the measurement light projected from the objective measurement optical system 10 is guided to the light receiving optical system 10b of the objective measurement optical system 10 through the concave surface mirror 85. Meanwhile, in the present example, a configuration in which the reflected light of the measurement light from the objective measurement optical system 10 is guided to the light receiving optical system 10b of the objective measurement optical system 10 through the concave surface mirror 85 has been described as an example, but the invention is not limited thereto. For example, a configuration may also be adopted in which the reflected light of the measurement light from the objective measurement optical system 10 does not go through the concave surface mirror 85.

In more detail, for example, in the present example, an optical axis between the concave surface mirror 85 and the subject eye E in the subjective measurement portion and an optical axis between the concave surface mirror 85 and the subject eye E in the objective measurement portion are configured as substantially the same axis. For example, in the present example, the optical axis L2 of the subjective measurement optical system 25 and the optical axis L1 of the objective measurement optical system 10 are combined with each other by the dichroic mirror 35, and are thus configured as the same axis.

### <Optical Path of Subjective Measurement Portion>

Hereinafter, the optical path of the subjective measurement portion will be described. For example, the subjective measurement portion reflects the target light flux having passed through the calibration optical system 60 in a direction of the subject eye by the concave surface mirror 85 to thereby guide the target light flux to the subject eye E, and forms an image of the target light flux having passed through the calibration optical system 60 in front of the subject eye E so as to optically have a predetermined examination distance. For example, at this time, the target light flux that has passed through the calibration optical system 60 passes through the optical path deviated from the optical axis L of the concave surface mirror 85, is incident on the concave surface mirror 85, is reflected to pass through the optical path deviated from the optical axis L of the concave surface mirror 85, and is guided to the subject eye E. For example, the examination visual target image seen from the examinee looks as if the visual target image is located farther than the actual distance between the subject eye E and the display 31. In other words, by using the concave surface mirror 85, it is possible to elongate the presenting distance of the visual target with respect to the subject eye E, and to present the examination visual target image to the examinee such that the image of the target light flux is seen at the predetermined examination distance.

A more detailed description will be given. In the following description, the left eye optical path will be described as an example, but also in the right eye optical path, the same configuration as the configuration of the left eye optical path is adopted. For example, in the left eye subjective measurement portion, the target light flux projected from the display 31 of the left eye measurement portion 7L is incident on the astigmatism calibration optical system 63 through the projection lens 33. The target light flux having passed through the astigmatism calibration optical system 63 is incident on the correction optical system 90 through the reflecting mirror 36, the dichroic mirror 35, the dichroic mirror 29, and the objective lens 14. The target light flux having passed through the correction optical system 90 is guided toward the left eye deflection mirror 81L from the left eye measurement portion 7L. The target light flux emitted from the left eye measurement portion 7L and reflected by the left eye deflection mirror 81 is reflected toward the concave surface mirror 85 by the reflecting mirror 84. For example, the target light flux emitted from the display 31 reaches the left subject eye EL through the optical member in this manner.

Accordingly, the examination visual target image calibrated by the calibration optical system 60 is formed on the fundus of the left subject eye EL based on a spectacle wearing position (for example, a position separated from the cornea apex position at approximately 12 mm) of the left subject eye EL. Therefore, this is equivalent to the arrangement of the astigmatism calibration optical system 63 in front of the eyes and the adjustment of a spherical power by a calibration optical system (in the present example, driving of the driving mechanism 39) of a spherical power, and thus the examinee can collimate the examination visual target image in a natural state through the concave surface mirror 85. Meanwhile, in the present example, the right eye optical path also has the same configuration as that of the left eye optical path, and the examination visual target image calibrated by a pair of left and right calibration optical systems 60 is formed on the fundi of both subject eyes, based on the spectacle wearing positions (for example, positions separated from the corneas apex positions at approximately 12 mm) of both the left subject eyes EL and the right subject eye ER. In this manner, the examinee responds to the examiner while looking straight at the examination visual target in a state of a natural sight, attempts calibration by the calibration optical system 60 until the examination visual target is seen properly, and subjectively measures the optical characteristics of the subject eye based on the calibration value thereof.

### <Optical Path of Objective Measurement Portion>

Subsequently, the optical path of the objective measurement portion will be described. In the following description, the left eye optical path will be described as an example, but also in the right eye optical path, the same configuration as the configuration of the left eye optical path is adopted. For example, in the objective measurement portion for the left eye, measurement light emitted from the light source 11 of the projection optical system 10a in the objective measurement optical system 10 is incident on the correction optical system 90 through the relay lens 12 to the objective lens 14. The measurement light having passed through the correction optical system 90 is projected toward the left eye deflection mirror 81L from the left eye measurement portion 7L. The measurement light emitted from the left eye measurement portion 7L and reflected by the left eye deflection mirror 81 is reflected toward the concave surface mirror 85 by the reflecting mirror 84. The measurement light reflected by the concave surface mirror reaches the left subject eye EL through the reflecting mirror 84, thereby forming a spot-shaped point light source image on the fundus of the left subject eye EL. At this time, a pupil projection image (projected luminous flux on the pupil) of the hole portion of the hole mirror 13 is eccentrically rotated at high speed by the prism 15 rotating around the optical axis.

Light of the point light source image formed on the fundus of the left subject eye EL is reflected and scattered, and is emitted to the subject eye E, is condensed by the objective lens 14 through the optical path through which the measurement light is transmitted, and passes through the dichroic mirror 29, the dichroic mirror 35, the prism 15, the hole mirror 13, the relay lens 16, and the mirror 17. The reflected light having passed through the components from the dichroic mirror to the mirror 17 is condensed again on the opening of the light receiving diaphragm 18, is converted into a substantially parallel luminous flux (a case of a normal vision eye) by the collimator lens 19, is extracted as a ring-shaped luminous flux by the ring lens 20, and is received by the image capture element 22 as a ring image. The received ring image is analyzed, and thus it is possible to objectively measure the optical characteristics of the subject eye E.

### <Control Portion>

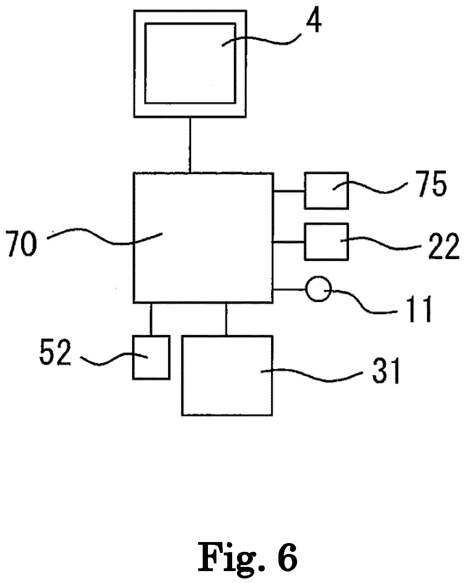

Fig. 6 is a view illustrating a control system of the subjective optometry apparatus 1 according to the present example. For example, various members, such as the monitor 4, the nonvolatile memory 75 (hereinafter, referred to as the memory 75), the light source 11 included in the measurement portion 7, the image capture element 22, the display 31, and the image capture element 52, are electrically connected to the control portion 70. Further, for example, the driving portions which are not illustrated and are included in the driving portion 9, the driving mechanism 39, the rotation mechanisms 62a and 62b, the driving portion 83, and the rotation mechanisms 92a and 92b respectively, are electrically connected to the control portion 70.

For example, the control portion 70 includes a CPU (processor), a RAM, a ROM, and the like. For example, the CPU controls each member of the subjective optometry apparatus 1. For example, the RAM temporarily stores various pieces of information. For example, various programs for controlling the operation of the subjective optometry apparatus 1, examination visual target data for various examinations, an initial value, and the like are stored in the ROM. Meanwhile, the control portion 70 may be configured with a plurality of control portions (that is, a plurality of processors).

For example, the memory 75 is a non-fugitive storage medium capable of holding stored contents even when the supply of power is stopped. For example, as the memory 75, a USB memory or the like which is attachably and detachably mounted to a hard disc drive, a flash ROM, and the subjective optometry apparatus 1, can be used. For example, a control program for controlling the subjective measurement portion and the objective measurement portion is stored in the memory 75.

### <Control Operation>

A control of the subjective optometry apparatus 1 including the above-described configuration will be described. For example, in the present example, the optical characteristics for a far distance of the subject eye E is measured by using the subjective optometry apparatus 1. It is needless to say that the optical characteristics for a near distance of the subject eye E may be measured by using the subjective optometry apparatus 1. For example, the examiner performs the objective measurement (objective optometry) and the subjective measurement (subjective optometry) with respect to the subject eye after positioning (aligning) the subjective optometry apparatus 1 and the subject eye E.

### <Alignment of Subject Eye>

The examiner instructs the examinee to place a jaw on a chin mount 5 and observe the fixation target displayed on the display 31 from the presentation window 3. The alignment index image is projected onto the subject eye E of the examinee as the light sources of the first index projection optical system 45 and the second index projection optical system 46 are turned on. In addition, the anterior ocular segment of subject eye E is detected by the anterior ocular segment image capture optical system 100. When the anterior ocular segment of the subject eye E is detected, the control portion 70 starts positioning the subject eye E and the measurement portion 7. In other words, the control portion 70 starts automatic alignment. In addition, for the details of automatic alignment, refer to, for example, JP-A-2017-86652.

### <Objective Measurement>

When the alignment of the subject eye E is completed, the control portion 70 displays the examination visual target (that is, the fixation target) for causing the subject eye E to be fixated on the display 31. For example, in the present example, the landscape visual target is displayed as such an examination visual target. It is needless to say that an examination visual target different from the landscape visual target may be displayed on the display 31.

For example, in the objective measurement, preliminary measurement of the objective eye refractive power may be performed with respect to the subject eye E. For example, the control portion 70 may move the display 31 in the optical axis L2 direction based on the result of the preliminary measurement, and may apply fogging to the subject eye E. In other words, after the display 31 is temporarily moved to a position where the subject eye E is in focus, the control portion 70 may apply cloudy fog to the subject eye E by moving the display 31 to a position where the amount of cloudy fog is appropriate. In addition, for the details of calculation of the amount of cloudy fog, it is desired to refer to, for example, JP-A-2017-99640.

In addition, for example, in the objective measurement, main measurement of the objective eye refractive power may be performed with respect to the subject eye E to which the cloudy fog is applied. For example, in the main measurement, the measurement image (that is, the above-described ring image) captured by the image capture element 22 is stored in the memory 75. For example, the control portion 70 obtains the eye refractive power in each longitudinal direction by analyzing the image of the ring image, and performs predetermined processing with respect to the eye refractive power, and accordingly, the optical characteristics (objective value) are acquired in the objective measurement of the subject eye E. In addition, the control portion 70 stores the acquired objective value in the memory 75.

In addition, the objective measurements may be performed for the left and right eyes at the same time, and may be performed at different timings for each of the left and right eyes.

### <Subjective Measurement>

When the subjective measurement is completed, the examiner operates the monitor 4 to switch the measurement mode and performs the subjective measurement with respect to the subject eye E. The control portion 70 controls at least any one of the calibration optical system 60 and the light projecting optical system 30 such that the eye refractive power of the subject eye E is calibrated to 0D based on the objective value of the subject eye E acquired by the objective measurement. In this case, the control portion 70 may calibrate at least any one of the cylindrical power and the astigmatic axis angle by rotating the cylindrical lenses 61a and 61b. Further, in this case, the control portion 70 may calibrate the spherical power by moving the display 31. According to this, it is possible to acquire the calibration power at which the eye refractive power of subject eye E becomes 0D. In addition, the control portion 70 may control at least any one of the calibration optical system 60 and the light projecting optical system 30 such that the eye refractive power of the subject eye E is calibrated to be a value other than 0D (for example, -1D or the like).

In addition, the control portion 70 also displays the examination visual target for measuring the eye refractive power of the subject eye on the display 31. For example, in the present example, the Landolt ring visual target of the required visual acuity value (for example, visual acuity value 1.0 or the like) is displayed as such an examination visual target. It is needless to say that an examination visual target different from the Landolt ring visual target may be displayed on the display 31.

The examiner selects a predetermined switch displayed on the monitor 4 in order to determine whether or not the calibration power set based on the optical characteristics in the objective measurement is appropriate for the examinee, and the examination visual target displayed on the display 31 is switched in accordance with the answer of the examinee. For example, the examiner switches the examination visual target to an examination visual target having a visual acuity value that is one step higher in a case where the answer of the examinee is a correct answer, and switches the examination visual target to an examination visual target having a visual acuity value that is one step lower in a case where the answer of the examinee is an incorrect answer. In other words, the control portion 70 may switch the examination visual target displayed on the display 31 based on a signal for changing the examination visual target from the monitor 4.

In addition, in a case where the above-described calibration power is not appropriate for the examinee, the examiner may operate the monitor 4 to change the calibration power of the calibration optical system 60 and the light projecting optical system 30, and may determine whether or not the calibration power after the change is appropriate for the examinee. For example, the control portion 70 acquires the calibration power in a case where the examiner determined that the calibration power is appropriate as the optical characteristics (subjective value) of the subject eye E in the subjective measurement. In addition, the control portion 70 stores the acquired subjective value in the memory 75.

In addition, the subjective measurements may be performed for the left and right subject eyes at the same time, and may be performed at different timings for each of the left and right subject eyes. In a case of different timings, the examination visual target may not be displayed on the display 31 on a non-measurement side, or fogging (for example, a constant refraction power is added to the objective eye refractive power) may be performed by the calibration optical system 60.

### <Angle-Of-View of Examination Visual Target>

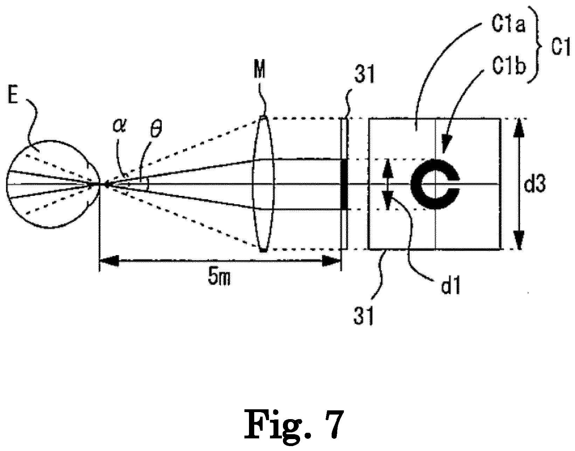

For example, in the above-described subjective measurement, the examination visual targets having various visual acuity values are projected onto the subject eye E. In the present example, an examination visual target C1 including a first examination visual target C1a and a second examination visual target C1b is displayed in a display region of the display 31 (refer to Fig. 7), and by changing the size of the second examination visual target C1b, it is possible to project the examination visual targets having visual acuity values onto the subject eye E. For example, for the examination visual target projected onto the subject eye E, the angle of view θ of the examination visual target is determined by the visual acuity value.

Fig. 7 is a view for describing an angle of view α of the display and an angle of view θ of the examination visual target. In Fig. 7, for the convenience, the description will be made by omitting the deflection mirror 81, the reflecting mirror 84, and the concave surface mirror 85, and by replacing the objective lens 14, the projection lens 33, and the projection lens 34 of the measurement portion 7 with one lens M. In addition, in Fig. 7 and Figs. 8 to 14 which will be described later, a state where the examination visual target C1 (first examination visual target C1a and second examination visual target C1b) is projected onto the fundus of the subject eye E is schematically illustrated. In addition, the examination visual target C1 has upper and lower ends and left and right ends, respectively, but in the present example, the left and right ends are omitted and only the upper and lower ends are illustrated in the description.

For example, the angle of view of the examination visual target C1 may be either an angle of view for the entire examination visual target or an angle of view for a part of the examination visual target. For example, in the present example, the angle of view for the entire examination visual target may be the angle of view for the first examination visual target C1a. In addition, for example, in the present example, the angle of view for a part of the examination visual target may be the angle of view for the second examination visual target C1b. In the following, a case where the angle of view of the examination visual target C1 is the angle of view for a part of the examination visual target is employed as an example.

For example, in the display region of the display 31, a background visual target having a white background is displayed as the first examination visual target C1a. It is needless to say that the background visual target may be a background visual target different from that of the present example. In addition, in the present example, the first examination visual target C1a is displayed in the entire display region of the display 31, but a part (for example, a region inside the display region by a predetermined number of pixels from the edge of the display region) of the display region of the display 31, the first examination visual target C1a may be displayed. In addition, for example, in the display region of the display 31, the Landolt ring visual target is displayed as the second examination visual target C1b.

For example, when the subject eye E gazes at the center of the examination visual target C1 displayed on the display 31, both ends (that is, the upper end and the lower end of the first examination visual target C1a) of the first examination visual target C1a appears in a peripheral visual field of the subject eye E. In the present example, since the first examination visual target C1a is displayed in the entire display region of the display 31, it is possible to consider that the upper end and the lower end of the display 31 appear in the peripheral visual field of the subject eye E. For example, a size d3 from the upper end and the lower end of the display 31 is projected onto the subject eye E with a predetermined size through each optical member (the lens M in Fig. 7) in the measurement portion 7. For example, the angle of view α of the display 31 is expressed as an angle formed by the center of the subject eye E (for example, the pupil center position of the subject eye E, and the like) and both ends of the display 31 projected onto the subject eye E.

In addition, for example, when the subject eye E gazes at the center of the examination visual target C1 displayed on the display 31, both ends (that is, the upper end and the lower end of the second examination visual target C1b) of the second examination visual target C1b appears in the peripheral visual field of the subject eye E. For example, the second examination visual target C1b displayed with a predetermined size d1 on the display 31 is projected onto the subject eye E with a predetermined size through each optical member in the measurement portion 7. For example, the angle of view θ of the second examination visual target C1b is expressed as an angle formed by the center of the subject eye E and both ends of the second examination visual target C1b projected onto the subject eye E.

For example, the angle of view θ of the examination visual target projected onto the subject eye E is determined for each visual acuity value of the second examination visual target C1b. As an example thereof, a case where the Landolt ring visual target having a visual acuity value of 0.5 is projected onto the subject eye E as the second examination visual target C1b when measuring the visual acuity for a far distance of the subject eye E, is employed as an example. For example, in this case, it is necessary to adjust the size (the size d1 of the second examination visual target C1b) of the Landolt ring visual target displayed on the display 31 such that the subject eye E is seen from the position where the distance (examination distance) between the subject eye E and the display 31 is optically 5 m apart when the gap of the Landolt ring visual target is approximately 3 mm and the size of the Landolt ring visual target is approximately 15 mm. For example, the angle of view θ for making the size of Landolt ring visual target seen at the subject eye E at approximately 15 mm is calculated to be approximately 0.17 degrees from a trigonometric function.

In addition, in the present example, the principal ray of the target light flux in the upstream optical path between the display 31 and the optical member (that is, the lens M in Fig. 7, the projection lens 33 or the projection lens 40 in Fig. 2) disposed to be the closest to the display 31 in the optical axis L2 direction is parallel (including approximately parallel) to the optical axis of the upstream optical path. In other words, the principal ray of the target light flux is telecentric (or substantially telecentric) on the object side (display 31 side) of the optical member disposed to be the closest to the display 31 in the optical axis L2 direction.

For example, the display 31 is moved in the optical axis L2 direction in accordance with the optical characteristics (eye refractive power) of the subject eye E. In a case where the principal ray of the target light flux is not parallel to the optical axis of the upstream optical path, even when the second examination visual target C1b is displayed on the display 31 with the predetermined size d1, the angle of view θ of the second examination visual target C1b projected (seen) onto the subject eye E changes for each eye refractive power of the subject eye E (details will be described later). For example, because the principal ray of the target light flux is parallel to the optical axis of the upstream optical path, it is possible to maintain the angle of view θ of the second examination visual target C1b projected onto the subject eye E to be the same regardless of the eye refractive power of the subject eye E.

### <Acquisition of Angle-Of-View Information>

For example, in the above-described subjective measurement, the control portion 70 acquires the angle-of-view information of the second examination visual target C1b. The angle-of-view information of the second examination visual target C1b may be information that can derive the angle of view θ of the second examination visual target C1b. For example, the angle-of-view information may be the angle of view θ of the examination visual target projected onto the subject eye E (for example, 0.17 degrees or the like). In addition, for example, the angle-of-view information may be the size (for example, 15 mm or the like) of the examination visual target projected onto the subject eye E. In this case, the control portion 70 can derive the angle of view θ from the examination distance from the subject eye E to the display 31 and the size of the acquired examination visual target. Further, for example, the angle-of-view information may be the size d1 of the second examination visual target C1b displayed on the display 31, the display region of the second exemption visual target C1b with respect to the display region of the display 31, and the like. For example, the control portion 70 can acquire the angle of view θ of the examination visual target projected onto the subject eye E by using the angle-of-view information.

For example, in the present example, a predetermined switch displayed on the monitor 4 is operated by the examiner, and the visual acuity value of the examination visual target projected onto the subject eye E is input, and accordingly, the angle of view θ of the examination visual target is obtained. As described above, the angle of view θ of the examination visual target projected onto the subject eye E is determined for each visual acuity value of the examination visual target. Therefore, for example, in the memory 75, the angle of view θ of the examination visual target that corresponds to each visual acuity value may be stored in advance as a table. Accordingly, the control portion 70 can acquire the angle of view θ of the examination visual target based on the visual acuity value input by the examiner.

### <Change in Projection Magnification>

For example, in the above-described subjective measurement, the the examination visual target in the first angle-of-view range (in the present example, the range of θ1 to θ2 which will be described later) is projected onto the subject eye E. In addition, for example, in the above-described subjective measurement, the control portion 70 controls the light projecting optical system 30, and accordingly, the examination visual target in the second angle-of-view range (in the present example, the range of θ3 to θ4 which will be described later) is projected onto the subject eye E. For example, the second angle-of-view range may be an angle-of-view range that exceeds the first angle-of-view range. In other words, in the above-described subjective measurement, the control portion 70 controls the light projecting optical system 30, and accordingly, the examination visual target in the second angle-of-view range that exceeds the first angle-of-view range that can be presented by the display 31 is projected onto the subject eye E. In addition, in the present example, as a second angle-of-view range, a case where the examination visual target in the angle-of-view range smaller than the first angle-of-view range is projected onto the subject eye E is employed as an example.

For example, the angle of view θ of the examination visual target that can be projected onto the subject eye E can be changed by changing the size d1 of the second examination visual target C1b displayed on the display 31. In this case, the angle of view α of the display 31 does not change, and the angle of view θ of the second examination visual target C1b changes. In addition, for example, the angle-of-view range of the examination visual target that can be presented to the subject eye E can be changed by changing the refractive power of the lens disposed in the light projecting optical system 30. In this case, both the angle of view α of the display 31 and the angle of view θ of the second examination visual target C1b change. This will be described hereinafter.

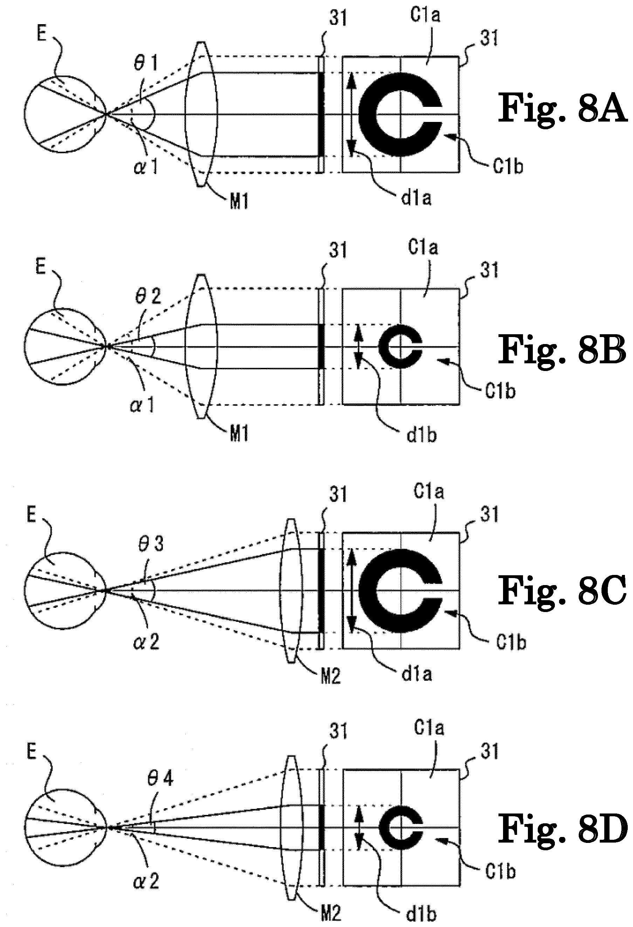

Figs. 8A to 8D are views illustrating a change in angle of view due to a change in projection magnification. In addition, in Figs. 8A to 8D, for the convenience, the description will also be made by omitting the deflection mirror 81, the reflecting mirror 84, and the concave surface mirror 85, and by replacing the objective lens 14, the projection lens 33 (or the projection lens 40), and the projection lens 34 of the measurement portion 7 with one lens. Further, the refractive power of the lens (hereinafter, referred to as a lens M1) illustrated in Figs. 8A and 8B is stronger than the refractive power of the lens (hereinafter, referred to as a lens M2) illustrated in Figs. 8C and 8D, and the refractive power of the lens M1 > the refractive power of the lens M2 is satisfied. For example, in the present example, the lens M1 is disposed in advance in the optical path, but the lens M1 is switched to the lens M2 in accordance with the angle of view θ acquired by the control portion 70. In other words, the refractive power of the light projecting optical system 30 changes in accordance with the angle of view θ acquired by the control portion 70.

For example, depending on the number of pixels and the pixel size of the display 31, the size of the second examination visual target C1b that can be displayed is limited. For example, in a case where a size d1a of the second examination visual target C1b illustrated in Fig. 8A is the largest size that can be displayed in the display region of the display 31, in a state where the lens M1 is disposed in the optical path, the angle of view θ1 of the examination visual target projected onto the subject eye E is the maximum angle of view. In addition, for example, in a case where a size d1b of the second examination visual target C1b illustrated in Fig. 8B is the smallest size that can be displayed in the display region of the display 31, in a state where the lens M1 is disposed in the optical path, the angle of view θ2 of the examination visual target projected onto the subject eye E is the minimum angle of view. In addition, for example, in a state where the lens M1 is disposed in the optical path, the display 31 is projected onto the subject eye E at an angle of view α1.

For example, in this manner, as the second examination visual target C1b displayed on the display 31 becomes greater, the angle of view becomes wider, and as the second examination visual target C1b displayed on the display 31 becomes smaller, the angle of view becomes narrower. In a state where the lens M1 is disposed in the optical path, the second examination visual target C1b in the range from the angle of view θ1 to the angle of view θ2 can be projected onto the subject eye E. For example, the control portion 70 changes the size of the second examination visual target C1b displayed on the display 31 based on the acquired angle of view of the examination visual target. Accordingly, the projection magnification of the light projecting optical system 30 is changed, and the examination visual target in the first angle-of-view range can be projected onto the subject eye E.

Here, for example, in a case where the acquired angle of view of the examination visual target is smaller than the angle of view θ2, the control portion 70 may change the projection magnification of the light projecting optical system 30 by switching the lens M1 disposed in the optical path to the lens M2.

For example, even when the size of the second examination visual target C1b displayed on the display 31 is the same in a state where the lens M1 is disposed in the optical path and a state where the lens M2 is disposed in the optical path, depending on the refractive power of the lens, the examination visual target is projected onto the subject eye E with different sizes of the different examination visual target. In other words, in a state where the lens M2 is disposed in the optical path, even when the second examination visual target C1b is displayed with the maximum size d1a that can be displayed in the display region of the display 31, the examination visual target is projected onto the subject eye E to be smaller than that in a state where the lens M1 is disposed in the optical path (refer to Figs. 8A and 8C). The maximum angle of view at this time is the angle of view θ3 of the examination visual target projected onto the subject eye E. In addition, in a state where the lens M2 is disposed in the optical path, even when the second examination visual target C1b is displayed with the minimum size d1b that can be displayed in the display region of the display 31, the examination visual target is projected onto the subject eye E to be smaller than that in a state where the lens M1 is disposed in the optical path (refer to Figs. 8B and 8D). The minimum angle of view at this time is the angle of view θ4 of the examination visual target projected onto the subject eye E. In addition, for example, in a state where the lens M2 is disposed in the optical path, the display 31 is projected onto the subject eye E at an angle of view α2.

For example, in a state where the lens M2 is disposed in the optical path, the second examination visual target C1b in the range from the angle of view θ3 to the angle of view θ4 can be projected onto the subject eye E. For example, the control portion 70 switches the lens (lens M1 in Figs. 8A and 8B and lens M2 in Figs. 8C and 8D) disposed in the light projecting optical system 30 based on the acquired angle of view of the examination visual target. In addition, for example, the control portion 70 changes the size of the second examination visual target C1b displayed on the display 31 based on the acquired angle of view of the examination visual target. Accordingly, the projection magnification of the light projecting optical system 30 is changed, and the examination visual target in the second angle-of-view range which is a angle-of-view range smaller than the first angle-of-view range can be projected onto the subject eye E.

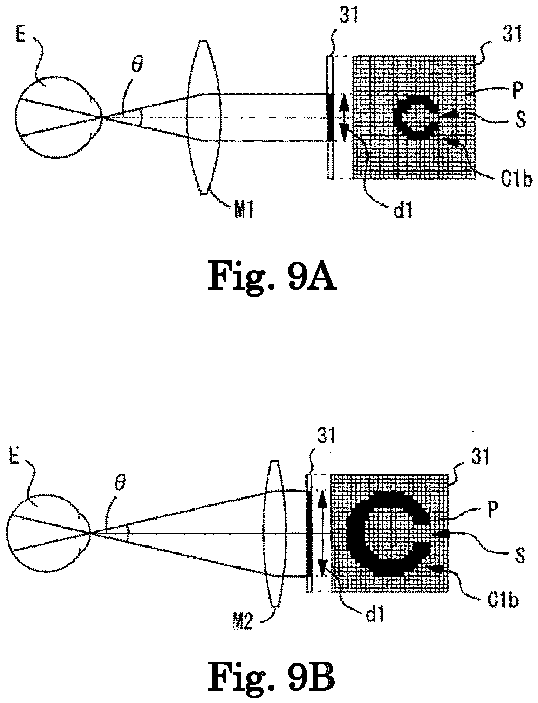

For example, in a case where the projection magnification of the light projecting optical system 30 is changed by switching between the lens M1 and the lens M2, even when the angle of view of the examination visual target projected onto the subject eye E is the same, it is possible to change the number of pixels that express the second examination visual target C1b displayed on the display 31. Accordingly, the number of pixels P in a gap of the Landolt ring visual target will be described as an example. Figs. 9A and 9B are views illustrating a change in the number of pixels due to a change in projection magnification. Fig. 9A is a view for describing the number of pixels when the second examination visual target C1b is displayed on the display 31 in a state where the lens M1 is disposed in the optical path. Fig. 9B is a view for describing the number of pixels when the second examination visual target C1b is displayed on the display 31 in a state where the lens M2 is disposed in the optical path. In Fig. 9A and Fig. 9B, the size d1 of the second examination visual target C1b displayed on the display 31 is different but the examination visual target is projected onto the subject eye E at the same angle of view θ. In addition, in Figs. 9A and 9B, the Landolt ring visual target is displayed as the second examination visual target C1b.

For example, in a state where the lens M1 is disposed in the optical path, when projecting the examination visual target having a certain angle of view θ onto the subject eye E, the second examination visual target C1b is displayed on the display 31 such that four pixels P enter a gap S of the Landolt ring visual target (refer to Fig. 9A). However, for example, in a state where the lens M2 is disposed in the optical path, when projecting the examination visual target having a certain angle of view θ onto the subject eye E, the second examination visual target C1b is displayed on the display 31 such that sixteen pixels P enter the gap S of the Landolt ring visual target (refer to Fig. 9B). For example, in this manner, when switching the lenses in the optical path, the number of pixels per angle of view θ of the examination visual target projected onto the subject eye E increases, and thus, the second examination visual target C1b displayed on the display 31 can be represented more finely.

For example, in the present example, in a state where the lens M1 is disposed in the optical path, although the number of pixels per angle of view θ becomes small, it becomes possible to project the examination visual target having a wide angle of view onto the subject eye E. In addition, for example, in the present example, in a state where the lens M2 is disposed in the optical path, although the number of pixels per angle of view θ becomes large, it becomes possible to project the examination visual target having a narrow angle of view onto the subject eye E.

### <Mode Setting based on Angle Of View of Examination Visual Target>

For example, the control portion 70 may set the first mode and the second mode respectively based on the acquired angle of view θ. For example, the first mode is a mode in which the examination visual target in the first angle-of-view range is projected onto the subject eye E, and in the examples illustrated in Figs. 8 and 9, the first mode is a mode in which the lens M1 is disposed in the optical path. In the present example, when the first mode is set, the projection lens 33 is disposed in the optical path of the light projecting optical system 30, and the examination visual target in the range of angles of view θ1 to θ2 can be projected onto the subject eye E. In addition, for example, the second mode is a mode in which the examination visual target in the second angle-of-view range is projected onto the subject eye E, and in the examples illustrated in Figs. 8 and 9, the second mode is a mode in which the lens M2 is disposed in the optical path. In the present example, when the second mode is set, the projection lens 40 is disposed in the optical path of the light projecting optical system 30, and the examination visual target in the range of angles of view θ3 to θ4 can be projected onto the subject eye E. In addition, for example, the control portion 70 drives the driving mechanism 43 based on the set mode. In other words, in accordance with the set mode, the control portion 70 switches the lens disposed in the optical path of the light projecting optical system 30 to the projection lens 33 or the projection lens 40.

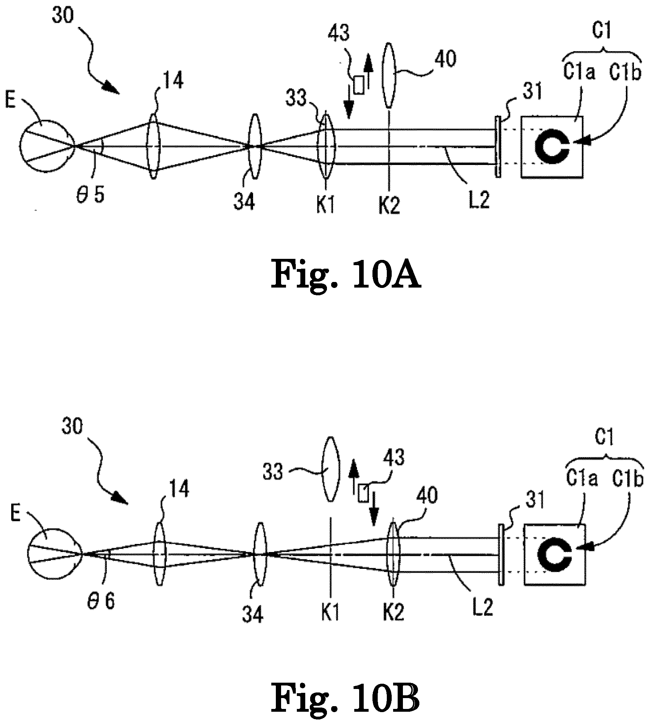

Figs. 10A and 10B are views for describing the switching of the projection lens 33 and the projection lens 40 that correspond to the mode. Fig. 10A illustrates a state where the first mode is set and the projection lens 33 is disposed in the optical path. Fig. 10B illustrates a state where the second mode is set and the projection lens 40 is disposed in the optical path. In addition, in Figs. 10A and 10B, for the convenience, only the objective lens 14, the projection lens 33, the projection lens 34, the projection lens 40, and the display 31 are illustrated, and other optical members are omitted.

For example, in the first mode, the projection lens 33 having a predetermined refractive power is disposed at a predetermined position K1 on the optical path. The predetermined position K1 may be a position where the target light flux parallel to the optical axis L2 among the target light flux emitted from the display 31 can be guided to the subject eye E. For example, when the angle of view obtained based on the visual acuity value input by the examiner is within the range (that is, within the first angle-of-view range) of the angle of view that can be handled by the projection lens 33, the control portion 70 projects the examination visual target having a angle of view θ5 onto the subject eye E by changing the size of the second examination visual target C1b displayed on the display 31.

In addition, for example, when the angle of view acquired based on the visual acuity value input by the examiner is a angle of view smaller than the range of the angle of view that can be handled by the projection lens 33, the control portion 70 may change the setting from the first mode to the second mode. In this case, the control portion 70 controls the driving mechanism 43 to remove the projection lens 33 from the optical path of the light projecting optical system 30, and disposes the projection lens 40 having a refractive power different from that of the projection lens 33 at a predetermined position K2 on the optical path. The predetermined position K2 may be a position where the target light flux parallel to the optical axis L2 among the target light flux emitted from the display 31 can be guided to the subject eye E. According to this, it becomes possible to project the examination visual target within the angle-of-view range (that is, within the second angle-of-view range) that can be handled by the projection lens 40 onto the subject eye E. For example, in the present example, the examination visual target of a angle of view θ6 is projected onto the subject eye E. It is needless to say that the control portion 70 may change the projection magnification of the light projecting optical system 30 by changing the size of the second examination visual target C1b displayed on the display 31, in addition to the switching of the lenses disposed in the optical path.

In addition, in the above-described description, the configuration in which the projection lens 33 is switched to the projection lens 40 has been employed as an example, but the present invention is not limited thereto. For example, the projection lens 34 may be switched to a lens having a refractive power different from that of the projection lens 34. In addition, for example, the objective lens 14 may be switched to a lens having a refractive power different from that of the objective lens 14. Even with the configurations, it is possible to change the projection magnification of the light projecting optical system 30 by switching between the first mode and the second mode.

As described above, for example, the subjective optometry apparatus in the present example projects the examination subject eye in the second angle-of-view range that exceeds the first angle-of-view range that can be presented to the subject eye by the visual target presenting portion onto the subject eye, by controlling the light projecting optical system. Therefore, it is possible to further widen the variable angle-of-view range of the examination visual target projected to the subject eye. In other words, it is possible to project the examination visual targets having various angles of view onto the subject eye.

In addition, for example, the subjective optometry apparatus in the present example projects the examination visual target in the second angle-of-view range onto the subject eye by moving the first optical member in the optical path of the light projecting optical system. Therefore, it is possible to change the angle-of-view range of the examination visual target projected onto the subject eye without providing a complicated configuration.

In addition, for example, in the subjective optometry apparatus in the present example, the principal ray of the target light flux in the upstream optical path between the visual target presenting portion and the second optical member disposed to be the closest to the visual target presenting portion in the optical axis direction of the optical path of the light projecting optical system is parallel to the optical axis of the upstream optical path. Accordingly, even when the eye refractive power of the subject eye changes, the examination visual target having the same angle of view can be projected onto the subject eye. In addition, it is possible to suppress occurrence of color unevenness or the like in the examination visual target.

### <Modified Example>

In addition, in the present example, as a configuration in which the optical members (the projection lens 33, the projection lens 40 and the like in the present example) is moved in the optical path of the light projecting optical system 30, a configuration in which the optical members disposed in the optical path are switched has been described as an example, but the invention is not limited thereto. For example, as the configuration in which the optical member is moved, a configuration in which the optical member is inserted into and removed from the optical path may be employed. In this case, at least any one of a configuration in which the optical member is inserted into the optical path, a configuration in which the optical member is removed from the optical path, a configuration in which the optical member is switched (exchanged), or the like may be used. In addition, for example, as the configuration in which the optical member is moved, the optical member may be moved in the optical axis direction of the optical path. In this case, the optical member may be configured to be moved parallel to the optical axis, or may be configured to be moved in an oblique direction. It is needless to say that the optical member may be configured to be moved in a direction different from the optical axis direction. For example, as the configuration in which the optical member is moved, a configuration combining the configuration in which the optical member is inserted into and removed from the optical path and the configuration in which the optical member is moved in the optical axis direction of the optical path may be employed. In addition, in the present example, each of the examination visual target in the first angle-of-view range and the examination visual target in the second angle-of-view range may be projected onto the subject eye E, and the optical member may be configured to be moved in the optical path of the optical system different from the light projecting optical system 30.

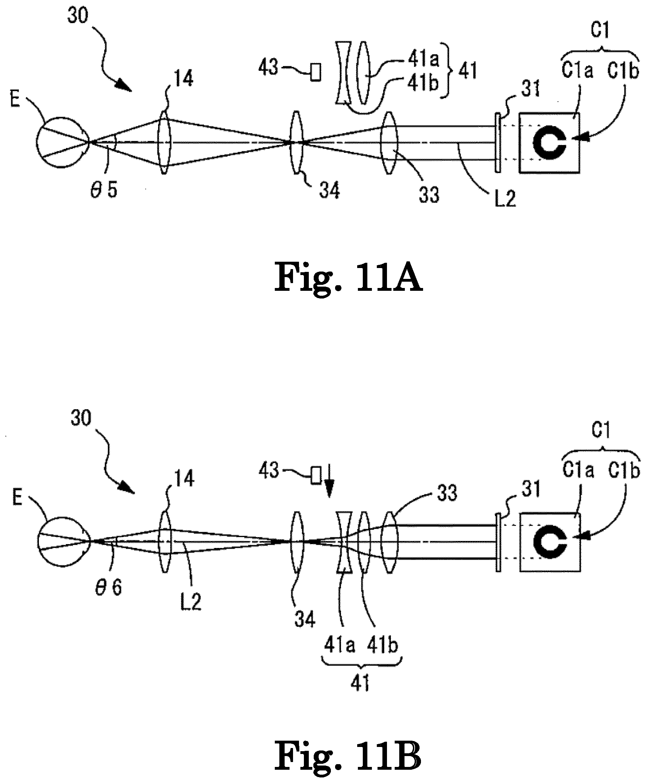

In addition, in the present example, the configuration in which the projection lens 33 and the projection lens 40 are switched in accordance with the set mode has been described as an example, but the invention is not limited thereto. For example, a lens 41 may be inserted into and removed from the optical path of the light projecting optical system 30 in accordance with the set mode. Figs. 11A and 11B are views for describing insertion and removal of the lens 41 that corresponds to the mode. Fig. 11A is a state where the first mode is set. Fig. 11B is a state where the second mode is set. For example, in the present example, the lens 41 is configured with a convex lens 41a and a concave lens 41b. It is needless to say that the lens 41 may be configured with a combination of lenses different from that of the present example.

For example, in the first mode, the lens 41 is not disposed in the optical path of the light projecting optical system 30, and the lens 41 is disposed out of the optical path of the light projecting optical system 30. Therefore, when the angle of view obtained based on the visual acuity value input by the examiner is within the first angle-of-view range, the control portion 70 can project the examination visual target within the first angle-of-view range onto the subject eye E by changing the size of the second examination visual target C1b displayed on the display 31. In addition, for example, when the angle of view acquired based on the visual acuity value input by the examiner is smaller than that in the first angle-of-view range, the control portion 70 changes the setting from the first mode to the second mode. In this case, the control portion 70 controls the driving mechanism 43 to insert the lens 41 into the optical path of the light projecting optical system 30. For example, in this state, the control portion 70 can project the examination visual target in the second angle-of-view range onto the subject eye E by changing the size of the second examination visual target C1b displayed on the display 31.

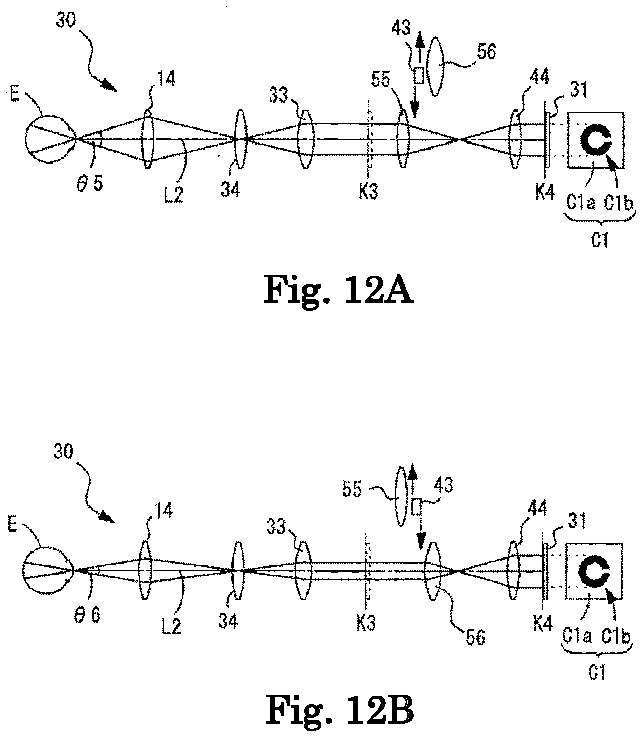

Further, in the present example, the light projecting optical system 30 may be a relay optical system and may form an intermediate image at an intermediate imaging position K3. Figs. 12A and 12B are views for describing a case where the light projecting optical system 30 is used as a relay optical system. Fig. 12A is a state where the first mode is set. Fig. 12B is a state where the second mode is set. For example, when the light projecting optical system 30 is a relay optical system, the predetermined position between the projection lens 33 and the display 31 may be set to the intermediate imaging position K3. Further, for example, when the light projecting optical system 30 is a relay optical system, a fixedly disposed lens 44 and a movable lens 55 or a lens 56 may be disposed between the intermediate imaging position K3 and the display 31. In addition, the lens 44 may be fixedly disposed at a position where the target light flux parallel to the optical axis L2 among the target light flux emitted from the display 31 can be guided to the subject eye E. Further, the refractive power of the lens 55 and the lens 56 is the lens 55 < the lens 56, and either one of the lens 55 and the lens 56 is disposed in the optical path by the driving mechanism 43 in accordance with the set mode.

For example, in the present example, in the first mode, the lens 55 is disposed in the optical path of the light projecting optical system 30, and in the second mode, the lens 55 is disposed in the optical path of the light projecting optical system 30. Accordingly, it is possible to change the projection magnification of the light projecting optical system 30 by switching between the first mode and the second mode. For example, the control portion 70 can project the examination visual target within the first angle-of-view range and the examination visual target within the second angle-of-view range onto the subject eye E by changing the size of the examination visual target C1b displayed on the display 31 in a state where each of the lenses is disposed in the optical path.

In addition, in a case where the light projecting optical system 30 is a relay optical system, in addition to the above-described configuration, the disposition position of the display 31 may be switched. In this case, the lens 44 and the lens 56 may be fixedly disposed between the intermediate imaging position K3 and the display 31. For example, in a case where the first mode is set, the display 31 is driven so as to be positioned at the intermediate imaging position K3. Accordingly, it is possible to project the examination visual target within the first angle-of-view range onto the subject eye E. Further, for example, in a case where the second mode is set, the display 31 is driven to a predetermined position (position K4 in Figs. 12A and 12B). Accordingly, it is possible to project the examination visual target within the second angle-of-view range onto the subject eye E.

In addition, although not illustrated in the present example, in the light projecting optical system 30, by moving at least two of the projection lens 33, the projection lens 34, and the objective lens 14 in the optical axis L2 direction, it is also possible to change the projection magnification of the light projecting optical system 30. As an example, in this case, when changing the setting from the first mode to the second mode, the projection lens 33 and the projection lens 34 may be moved away from the display 31.

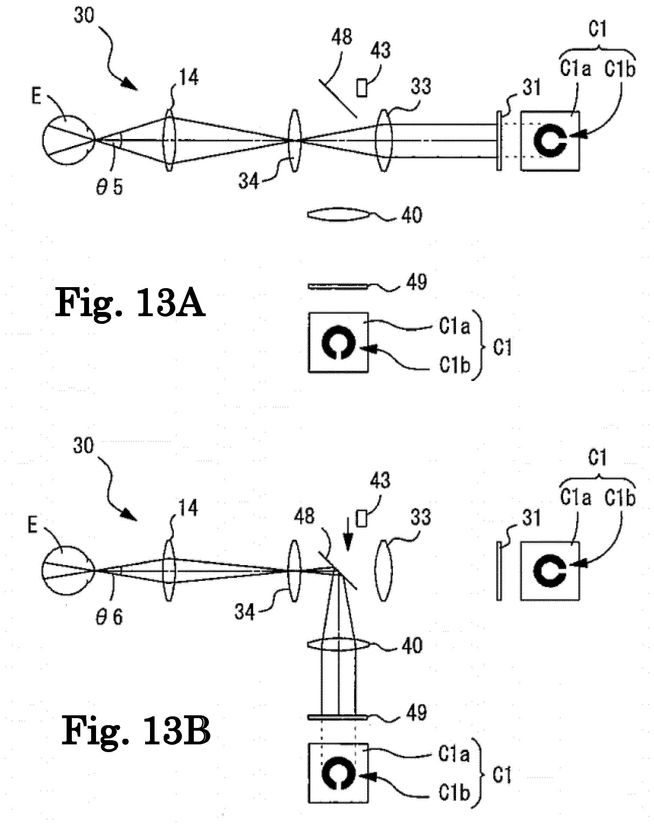

In addition, in the present example, the configuration in which the lens is driven as the optical member and the projection magnification is switched has been described as an example, but the invention is not limited thereto. For example, the mirror may be inserted into and removed from the optical path of the light projecting optical system 30. Figs. 13A and 13B are views for describing the insertion and removal of the mirror that corresponds to the mode. Fig. 13A is a state where the first mode is set. Fig. 13B is a state where the second mode is set. For example, in a case of inserting or removing the mirror, the projection lens 40 and the display 49 may be disposed in the optical path to which the target light flux reflected by the mirror is directed. For example, in the first mode, the mirror 48 is not disposed in the optical path of the light projecting optical system 30, and the mirror 48 is disposed out of the optical path of the light projecting optical system 30. Therefore, the target light flux (hereinafter, first target light flux) emitted from the display 31 reaches the subject eye E via the projection lens 33, the projection lens 34, and the objective lens 14. In other words, it is possible to project the examination visual target within the angle-of-view range (that is, within the first angle-of-view range) of the angle of view that can be handled by the projection lens 33 onto the subject eye E. In addition, for example, in the second mode, the mirror 48 is disposed by the driving mechanism 43 in the optical path of the light projecting optical system 30. Therefore, the target light flux (hereinafter, second target light flux) emitted from the display 49 is reflected by the mirror 48 via the projection lens 40 and further reaches the subject eye E via the projection lens 34 and the objective lens 14. In other words, it is possible to project the examination visual target within the angle-of-view range (that is, within the second angle-of-view range) of the angle of view that can be handled by the projection lens 40 onto the subject eye E.

In addition, in the above-described description, the mirror disposed in the optical path of the light projecting optical system 30 may be a half mirror. With such a configuration, it is possible to guide both the first target light flux and the second target light flux to the subject eye E without inserting and removing the mirror into and from the optical path. Accordingly, among the examination visual targets projected onto the subject eye E, a region (for example, a periphery portion of the examination visual target) having a wide angle of view can be expressed as the first target light flux and a region (for example, the center portion of the examination visual target) having a narrow angle of view can be expressed as the second target light flux.

In addition, in the present example, a configuration in which an optical member (hereinafter, a first optical member) moved in the optical path of the light projecting optical system 30 and an optical member (hereinafter, a second optical member) disposed to be the closest to the display 31 in the optical axis L2 direction are commonly used, and the second optical member is movable has been described as an example, but the invention is not limited thereto. For example, the second optical member may be an optical member fixedly disposed in the optical path of the light projecting optical system 30. In other words, each of the first optical member and the second optical member may be a different optical member.

In addition, in the present example, a configuration in which the principal ray of the target light flux in the upstream optical path between the display 31 and the optical member (for example, the projection lens 33, the projection lens 40, and the like) disposed to be the closest to the display 31 in the optical axis L2 direction is parallel to the optical axis of the upstream optical path has been described. In a comparative example, the principal ray of the target light flux in the upstream optical path between the display 31 and the optical member disposed to be the closest to the display 31 in optical axis L2 direction is not parallel to the optical axis of the upstream optical path. In this case, the angle of view θ of the second examination visual target C1b projected onto the subject eye E changes for each eye refractive power of the subject eye E.

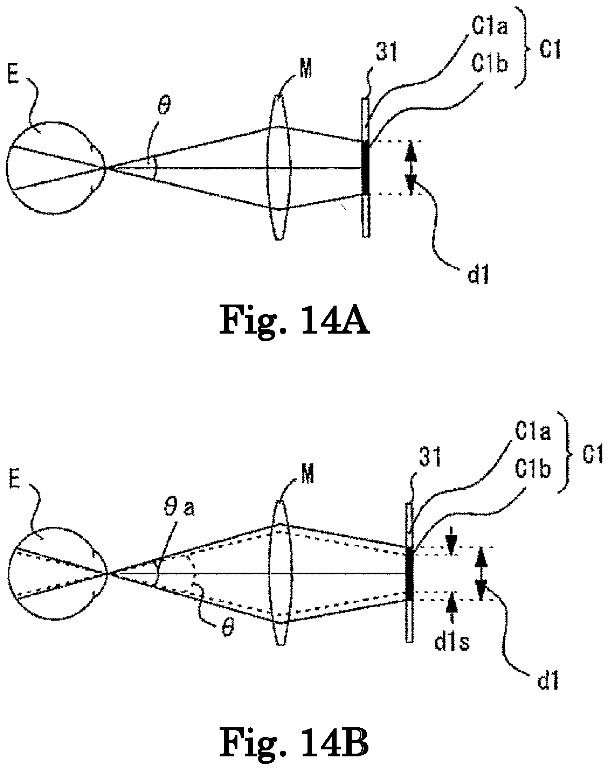

Figs. 14A and 14B are views for describing a comparative example case where the principal ray of the target light flux is not parallel to the optical axis. Fig. 14A illustrates the subject eye E (for example, the subject eye of -1 D) of which the eye refractive power is weak. Fig. 14B illustrates the subject eye E (for example, the subject eye of -15 D) of which the eye refractive power is weak. For example, when the display 31 is moved in the optical axis direction in accordance with the eye refractive power of the subject eye E, the examination visual targets projected onto the subject eye E have different sizes even when the second examination visual target C1b is displayed with the predetermined size d1. For example, in Fig. 14A, the second examination visual target C1b displayed with the size d1 onto the display 31 is projected onto the subject eye E at the angle of view θ. However, for example, in Fig. 14B, as illustrated by the solid line, the second examination visual target C1b displayed with the size d1 on the display 31 is projected onto the subject eye E at the angle of view θ.

Therefore, in such an example, the control portion 70 may perform the correction processing such that the examination visual target is projected at the same angle onto both the subject eye E of which the eye refractive power is weak as illustrated in Fig. 14A and the subject eye E of which the eye refractive power is strong as illustrated in Fig. 14B. For example, the control portion 70 may not change the angle of view of the examination visual target projected onto the subject eye E by the eye refractive power of the subject eye E by correcting the size of the second examination visual target C1b displayed on the display 31 in advance. For example, the memory 75 may store a correction amount for correcting the angle of view of the examination visual target projected onto the subject eye E for each eye refractive power of the subject eye E. For example, by controlling the display on the display 31 based on the correction amount, the control portion 70 can maintain the angle of view of the examination visual target projected onto the subject eye E to be the same. For example, in the present example, by displaying the second examination visual target C1b in advance with the size d1s on the display 31, the examination visual target is projected onto the subject eye E with the same angle of view θ.

In addition, in a case where the light projecting optical system 30 is an optical disposition that guides the target light flux not parallel to the optical axis L2 to the subject eye E, there is a case where the color temperature or the luminance of the examination visual target projected onto the subject eye E is uneven. In other words, the color tone changes or the luminance appears to be lower as the periphery portion of the examination visual target projected onto the subject eye E changes. Therefore, the control portion 70 may control the display on the display 31 in advance in consideration of such unevenness.

In addition, for example, in a case of correcting the size of the second examination visual target C1b by controlling the display on the display 31 as described above, the correction amount of the subject eye having a stronger eye refractive power than that of the subject eye having a weak eye refractive power becomes large. In other words, for example, since the examination visual target of the same angle of view θ is projected onto the subject eye E, it is necessary to reduce the size of the second examination visual target C1b displayed on the display 31 to be smaller than the subject eye having a weak eye refractive power. Therefore, for the subject eye having a strong eye refractive power, even when the angle of view θ is large, the smallest size that can be displayed in the display region of the display 31 is achieved, and there is a case where it is not possible to handle the angle of view of the visual acuity value input by the examiner. In this case, the control portion 70 may change the projection magnification of the light projecting optical system 30 by further moving the optical member in the optical path of the light projecting optical system 30. As an example, a configuration in which the projection lens 40 is exchanged for a lens having a stronger refractive power or is added by a separate lens in the optical path may be employed.

In addition, in the present example, the configuration in which the first mode for projecting the examination visual target in the first angle-of-view range onto the subject eye E, the second mode for projecting the examination visual target in the second angle-of-view range onto the subject eye E are provided has been described as an example, but the invention is not limited thereto. For example, a configuration in which two or more modes can be set may be employed. In this case, in addition to the first angle-of-view range (for example, within the range of angle of view ±20 degrees and the like) or the second angle-of-view range (for example, within the range of angle of view ±3 degrees and the like), a mode in which the examination visual target of the third angle-of-view range (for example within the range of angle of view ±10 degrees and the like) may be provided as the moderate angle of view.

Further, in the present example, in order to project the examination visual target having a smaller angle of view θ as the second angle-of-view range onto the subject eye E, a configuration in which the lens is moved in the optical path of the light projecting optical system 30 and the projection magnification is switched has been described as an example, but the invention is not limited thereto. For example, in order to project the examination visual target having the greater angle of view θ as the second angle-of-view range onto the subject eye E, the lens may be driven in the optical path of the light projecting optical system 30 and the projection magnification may be switched. For example, in this case, the concave lens may be driven in the optical path of the light projecting optical system 30.

In addition, in the present example, the configuration in which, in accordance with the visual acuity value input by the examiner, the angle of view of the examination visual target projected onto the subject eye E is acquired and a mode for changing the projection magnification is set based on the acquired angle of view has been described as an example, but the invention is not limited thereto. For example, a mode for changing the projection magnification may be configured to be selectable by the examiner and to be manually performed.

In addition, in the present example, the configuration in which the second examination visual target C1b is displayed on the display 31 in order to project the examination visual target onto the subject eye E has been described as an example, but the invention is not limited thereto. For example, by using the light source and the visual target plate (for example, a glass plate that forms the second examination visual target C1b, and the like) for presenting the second examination visual target C1b to the subject eye E, the examination visual target may be projected onto the subject eye E. Accordingly, it is possible to maintain the angle of view θ, and to project a smaller examination visual target onto the subject eye E.

In addition, in the present example, the configuration in which the angle of view of the examination visual target C1 is a angle of view for a part of the examination visual target and the angle of view of the second examination visual target C1b is set has been described as an example, but the invention is not limited thereto. For example, the angle of view of the examination visual target C1 may be set as the angle of view for the entire examination visual target, and the angle of view of the first examination visual target C1a may be set. As an example, at this time, a configuration in which the landscape visual target is displayed as the first examination visual target C1a in the display region of the display 31, and the second examination visual target C1b is not displayed may be employed. For example, in such a case, the angle formed by the center of the subject eye E and both ends of the first examination visual target C1a projected onto the subject eye E may be set as the angle of view θ.

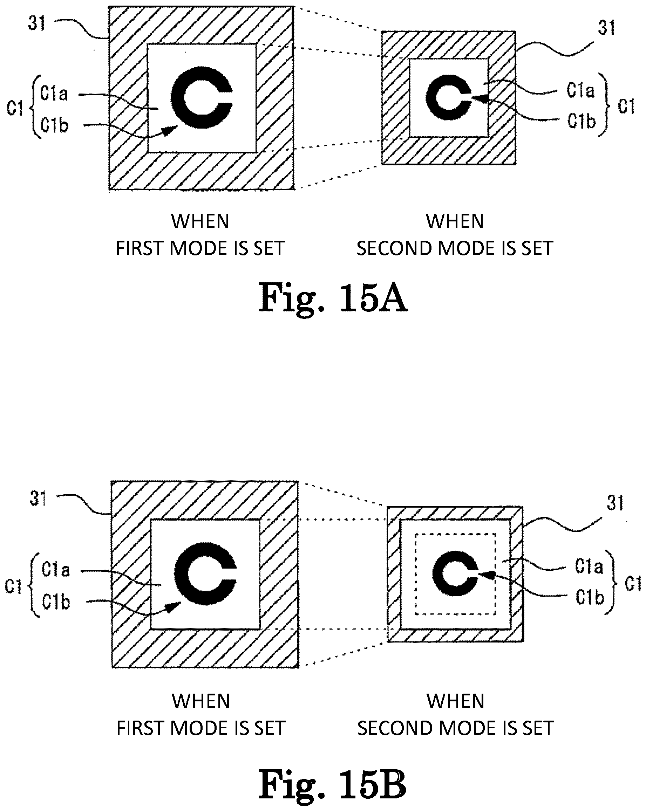

In addition, for example, when switching from the first mode to the second mode in the middle of the subjective measurement (or when switching from the second mode to the first mode in the middle of the subjective measurement), there is a case where not only the second examination visual target C1b is projected to be small (or large) but also the first examination visual target C1a is projected to be smaller (or greater) onto the subject eye. Figs. 15A and 15B are views for describing a change in appearance of the examination visual target C1 due to the switching of the mode. Fig. 15A illustrates a case where the display of the display 31 is not controlled. Fig. 15B illustrates a case where the display of the display 31 is not controlled. In addition, in Figs. 15A and 15B, in a region inside the display region by a predetermined number of pixels from the edge of the display region of the display 31, the first examination visual target C1a (for example, a background target of a white background) and the second examination visual target C1b (for example, Landolt ring visual target) are displayed.

For example, in the present example, when switching from the first mode to the second mode in the middle of the subjective measurement, each of the first examination visual target C1a and the second examination visual target C1b is seen to the subject eye E being reduced (refer to Fig. 15A). Here, the control portion 70 may control the display of the display 31 such that the first examination visual target C1a is projected onto the subject eye E having the same size even when the mode is switched. In other words, the display of the display 31 may be controlled such that the angle of view of the first examination visual target C1a does not change even when the mode is switched. For example, in the present example, the display region of the first examination visual target C1a with respect to the display region of the display 31 is changed (refer to Fig. 15B). Accordingly, even when the mode is switched in the middle of the subjective measurement, only the second examination visual target C1b can be projected to be small onto the subject eye E.
1 subjective optometry apparatus
7 measurement portion
10 objective measurement optical system
25 subjective measurement optical system
30 light projecting optical system
60 calibration optical system
70 control portion
75 memory
81 deflection mirror
84 reflecting mirror
85 concave surface mirror
90 calibration optical system
100 anterior ocular segment image capture optical system

## Claims

1. A subjective optometry apparatus (1) for subjectively measuring optical characteristics of a subject eye (E), comprising:
a light projecting optical system (30) that has a visual target presenting portion (31) for emitting a target light flux that presents a visual target (C1a, C1b) to the subject eye (E) in a first angle-of-view range, and guides the target light flux emitted from the visual target presenting portion (31) to the subject eye (E); and
a control portion (70) that controls the light projecting optical system (30) to project a visual target (C1a, C1b) presented by the target light flux emitted from the visual target presenting portion (31) onto the subject eye (E) in a second angle-of-view range that exceeds the first angle-of-view range, wherein
a principal ray of the target light flux in a upstream optical path between the visual target presenting portion (31) and a second optical member (40) disposed to be the closest to the visual target presenting portion (31) in an optical axis direction (L2) of an optical path of the light projecting optical system (30) is parallel to an optical axis of the upstream optical path.

2. The subjective optometry apparatus (1) according to claim 1,
wherein the control portion (70) drives a driving portion to move a first optical member (33) in an optical path of the light projecting optical system (30) so as to project the visual target (C1a, C1b) onto the subject eye (E) in the second angle-of-view range.

3. The subjective optometry apparatus (1) according to claim 2,
wherein the control portion (70) drives the driving portion to insert the first optical member (33) into the optical path or to remove the first optical member (33) from the optical path so as to project the visual target (C1a, C1b) onto the subject eye (E) in the second angle-of-view range.

4. The subjective optometry apparatus (1) according to claim 2 or 3,
wherein the control portion (70) drives the driving portion to move the first optical member (33) in an optical axis direction (L2) of the optical path.

5. The subjective optometry apparatus (1) according to any one of claims 1 to 4, further comprising:
a setting portion that sets either a first mode for projecting the visual target (C1a, C1b) in the first angle-of-view range or a second mode for projecting the visual target in the second angle-of-view range,
wherein the control portion (70) drives the driving portion based on a mode set by the setting portion.

6. The subjective optometry apparatus (1) according to any one of claims 1 to 5,
wherein the control portion (70) changes a projection magnification of the light projecting optical system (30) to project the visual target (C1a, C1b) onto the subject eye (E) in the second angle-of-view range.

7. The subjective optometry apparatus (1) according to any one of claims 1 to 6, further comprising:
a calibration optical system (60) that is disposed in an optical path of the light projecting optical system (30) and changes optical characteristics of the target light flux; and
a subjective measurement portion (25) that subjectively measures the optical characteristics of the subject eye (E).

## Patentansprüche

1. Subjektive Optometrievorrichtung (1) zum subjektiven Messen optischer Eigenschaften eines Subjektauges (E), umfassend:
ein optisches Lichtprojektionssystem (30), das einen visuellen Zielpräsentationsabschnitt (31) zum Emittieren eines Ziellichtflusses aufweist, der dem Subjektauge (E) in einem ersten Betrachtungswinkelbereich ein visuelles Ziel (C1a, C1b) präsentiert, und den von dem visuellen Zielpräsentationsabschnitt (31) emittierten Ziellichtfluss zu dem Subjektauge (E) leitet; und
einen Steuerabschnitt (70), der das optische Lichtprojektionssystem (30) steuert, um ein visuelles Ziel (C1a, C1b), das durch den von dem visuellen Zielpräsentationsabschnitt (31) emittierten Ziellichtfluss präsentiert wird, auf das Subjektauge (E) in einem zweiten Betrachtungswinkelbereich zu projizieren, der den ersten Betrachtungswinkelbereich überschreitet, wobei
ein Hauptstrahl des Ziellichtflusses in einem stromaufwärtigen optischen Pfad zwischen dem visuellen Zielpräsentationsabschnitt (31) und einem zweiten optischen Element (40), das angeordnet ist, um dem visuellen Zielpräsentationsabschnitt (31) in einer optischen Achsenrichtung (L2) eines optischen Pfads des optischen Lichtprojektionssystems (30) am nächsten zu sein, parallel zu einer optischen Achse des stromaufwärtigen optischen Pfads ist.

2. Subjektive Optometrievorrichtung (1) nach Anspruch 1,
wobei der Steuerabschnitt (70) einen Antriebsabschnitt antreibt, um ein erstes optisches Element (33) in einem optischen Pfad des optischen Lichtprojektionssystems (30) zu bewegen, um das visuelle Ziel (C1a, C1b) auf das Subjektauge (E) in dem zweiten Betrachtungswinkelbereich zu projizieren.

3. Subjektive Optometrievorrichtung (1) nach Anspruch 2,
wobei der Steuerabschnitt (70) den Antriebsabschnitt antreibt, um das erste optische Element (33) in den optischen Pfad einzuführen oder das erste optische Element (33) aus dem optischen Pfad zu entfernen, um das visuelle Ziel (C1a, C1b) auf das Subjektauge (E) in dem zweiten Betrachtungswinkelbereich zu projizieren.

4. Subjektive Optometrievorrichtung (1) nach Anspruch 2 oder 3,
wobei der Steuerabschnitt (70) den Antriebsabschnitt antreibt, um das erste optische Element (33) in einer optischen Achsenrichtung (L2) des optischen Pfads zu bewegen.

5. Subjektive Optometrievorrichtung (1) nach einem der Ansprüche 1 bis 4, ferner umfassend:
einen Einstellabschnitt, der entweder einen ersten Modus zum Projizieren des visuellen Ziels (C1a, C1b) in dem ersten Betrachtungswinkelbereich oder einen zweiten Modus zum Projizieren des visuellen Ziels in dem zweiten Betrachtungswinkelbereich einstellt,
wobei der Steuerabschnitt (70) den Antriebsabschnitt basierend auf einem durch den Einstellabschnitt eingestellten Modus antreibt.

6. Subjektive Optometrievorrichtung (1) nach einem der Ansprüche 1 bis 5,
wobei der Steuerabschnitt (70) eine Projektionsvergrößerung des optischen Lichtprojektionssystems (30) ändert, um das visuelle Ziel (C1a, C1b) auf das Subjektauge (E) in dem zweiten Betrachtungswinkelbereich zu projizieren.

7. Subjektive Optometrievorrichtung (1) nach einem der Ansprüche 1 bis 6, ferner umfassend:
ein optisches Kalibrierungssystem (60), das in einem optischen Pfad des optischen Lichtprojektionssystems (30) angeordnet ist und optische Eigenschaften des Ziellichtflusses ändert; und
einen subjektiven Messabschnitt (25), der die optischen Eigenschaften des Subjektauges (E) subjektiv misst.

## Revendications

1. Appareil d'optométrie subjective (1) pour mesurer subjectivement des caractéristiques optiques d'un oeil d'un sujet (E), comprenant :
un système optique de projection de lumière (30) qui a une partie de présentation de cible visuelle (31) pour émettre un flux de lumière cible qui présente une cible visuelle (C1a, C1b) à l'oeil du sujet (E) dans une première plage d'angle de vue, et guide le flux de lumière cible émis par la partie de présentation de cible visuelle (31) vers l'oeil du sujet (E) ; et
une partie de commande (70) qui commande le système optique de projection de lumière (30) pour projeter une cible visuelle (C1a, C1b) présentée par le flux de lumière cible émis par la partie de présentation de cible visuelle (31) sur l'oeil du sujet (E) dans une seconde plage d'angle de vue qui dépasse la première plage d'angle de vue, dans lequel
un rayon principal du flux de lumière cible dans un chemin optique en amont entre la partie de présentation de cible visuelle (31) et un second élément optique (40) disposé pour être le plus proche de la partie de présentation de cible visuelle (31) dans une direction d'axe optique (L2) d'un chemin optique du système optique de projection de lumière (30) est parallèle à un axe optique du chemin optique en amont.

2. Appareil d'optométrie subjective (1) selon la revendication 1,
dans lequel la partie de commande (70) entraîne une partie d'entraînement pour déplacer un premier élément optique (33) dans un chemin optique du système optique de projection de lumière (30) de manière à projeter la cible visuelle (C1a, C1b) sur l'oeil du sujet (E) dans la seconde plage d'angle de vue.

3. Appareil d'optométrie subjective (1) selon la revendication 2,
dans lequel la partie de commande (70) entraîne la partie d'entraînement pour insérer le premier élément optique (33) dans le chemin optique ou pour retirer le premier élément optique (33) du chemin optique de manière à projeter la cible visuelle (C1a, C1b) sur l'oeil du sujet (E) dans la seconde plage d'angle de vue.

4. Appareil d'optométrie subjective (1) selon la revendication 2 ou 3,
dans lequel la partie de commande (70) entraîne la partie d'entraînement pour déplacer le premier élément optique (33) dans une direction d'axe optique (L2) du chemin optique.

5. Appareil d'optométrie subjective (1) selon l'une quelconque des revendications 1 à 4, comprenant en outre :
une partie de réglage qui règle soit un premier mode pour projeter la cible visuelle (C1a, C1b) dans la première plage d'angle de vue, soit un second mode pour projeter la cible visuelle dans la seconde plage d'angle de vue,
dans lequel la partie de commande (70) entraîne la partie d'entraînement sur la base d'un mode réglé par la partie de réglage.

6. Appareil d'optométrie subjective (1) selon l'une quelconque des revendications 1 à 5,
dans lequel la partie de commande (70) change un grossissement de projection du système optique de projection de lumière (30) pour projeter la cible visuelle (C1a, C1b) sur l'oeil du sujet (E) dans la seconde plage d'angle de vue.

7. Appareil d'optométrie subjective (1) selon l'une quelconque des revendications 1 à 6, comprenant en outre :
un système optique d'étalonnage (60) qui est disposé dans un chemin optique du système optique de projection de lumière (30) et change des caractéristiques optiques du flux de lumière cible ; et
une partie de mesure subjective (25) qui mesure subjectivement les caractéristiques optiques de l'oeil du sujet (E).
